# EUROPEAN PATENT APPLICATION

(11) **EP 2 481 755 A1**
(43) Date of publication of application: **01.08.2012**
(21) Application number: 10818832.7
(22) Date of filing: 24.09.2010
(51) Int. Cl.: C07K 16/28, A61K 39/395, A61P 35/00, A61P 35/02, A61P 43/00, C07K 16/46, C12N 15/09

(54) **ANTIBODY CAPABLE OF RECOGNIZING HLA CLASS I**

(30) Priority: 24.09.2009 JP 2009218677
(71) Applicant: Chugai Seiyaku Kabushiki Kaisha, Kita-ku Tokyo 115-8543 (JP)
(72) Inventor: KIMURA, Naoki, Gotemba-shi Shizuoka 412-8513 (JP); HABU, Kiyoshi, Gotemba-shi Shizuoka 412-8513 (JP)
(74) Representative: Vossius & Partner
(86) International application number: PCT/JP2010/066494
(87) International publication number: WO 2011/037160

(57) **Abstract**

An objective of the present invention is to provide antibodies that recognize HLA class I and have significant cell death-inducing activity. To achieve the above objective, first, the present inventors immunized mice with soluble HLA-A to which a FLAG tag is attached. Four days after the final immunization, spleen cells from the mice were fused with mouse myeloma cells. Then, hybridoma screening was carried out using the cell aggregation-inducing activity and cell death-inducing activity as an index. Thus, the monoclonal antibody F17B1 that has strong cell death-inducing activity was selected. The sequences of the H chain and L chain variable regions of the mouse monoclonal antibody F17B1 were determined to humanize this antibody. Then, the humanized anti-HLA class I antibody H2-G1d_L1-k0 was assessed for cell death induction. The result showed that H2-G1d_L1-k0 suppressed the growth of the IM9 cell line in a concentration dependent manner.

## Description

### Technical Field

The present invention relates to HLA class I-recognizing antibodies.

### Background Art

HLA, an important immune response molecule, is involved in recognizing and eliminating exogenous antigens, bacteria, virus-infected cells, and other such foreign substances. The main role of the HLA class I molecule is to present antigenic peptides (made up of about eight to ten amino acid residues) produced inside cells to CD8⁺ T cells. Accordingly, the HLA molecule plays a very important role in the immune response, and in immune tolerance induced by peptide presentation. HLA molecules are categorized into class I and class II. Class I molecules form a heterodimer made up of a 12-KD β2 microglobulin (β2M) and a 45-KD α-chain composed of three domains, α1-3. Class II molecules form a heterodimer made up of a 30-34 KD α-chain composed of two domains, α1 and α2, and a 26-29 KD β-chain composed of two domains, [β1 and β2. HLA class I molecules are known to be further classified into HLA-A, B, C, and such.

To date, cell growth-suppressing and cell death-inducing effects of anti-HLA class I antibodies have been reported for lymphocytes, suggesting that HLA class I molecules may be signal transduction molecules. For example, it has been reported that cell growth of activated lymphocytes is suppressed by the B9.12.1 antibody against the α1 domain of HLA class I, W6/32 antibody against the α2 domain, and TP25.99 and A1.4 antibodies against the α3 domain (Fayen et al., Int. Immunol., 10: 1347-1358 (1998); Genestier et al., Blood, 90: 3629-3639 (1997)). In addition, two antibodies against the αβ1 domain, MoAb90 and YTH862, have been reported to induce apoptosis in activated lymphocytes (Genestier et al., Blood, 90: 3629-3639 (1997); Genestier et al., Blood, 90: 726-735 (1997); Genestier et al., J. Biol. Chem., 273: 5060-5066 (1998)). Apoptosis induced by these two antibodies has been shown to be a caspase-mediated reaction (Genestier et al., J. Biol. Chem., 273: 5060-5066 (1998)). Accordingly, there is speculation that HLA class I expressed in lymphocytes is involved in apoptosis signal transduction.

Furthermore, 5H7 antibody against the α3 domain of HLA class I (Woodle et al., J. Immunol., 158: 2156-2164 (1997)), and the RE2 antibody against the α2 domain of mouse MHC class I (Matsuoka et al., J. Exp. Med., 181: 2007-2015 (1995)) have also been reported to induce cell death in activated lymphocytes and the like.

The monoclonal antibody 2D7 (Oka, T., "Sankyo Seimei-kagaku-zaidan Kenkyu Hokoku-shu (Research Reports of the Sankyo Life Science Foundation)" (1998) 12: 46-56), obtained by immunizing human myeloma cells, is also reported to be an HLA class I-recognizing antibody. More particularly, when made into a low-molecular-weight antibody (diabody), it can quickly induce cell death in human myeloma cells. Furthermore, the monoclonal antibody C3B3, obtained by immunizing mice with cells coexpressing HLA-A and human α2M molecules (WO 2008/007755), is an antibody that recognizes HLA class I antigen α2 domain; it also shows strong cytotoxic activity when cross-linked with an anti-mouse IgG antibody. Furthermore, when modified into a low-molecular-weight antibody (C3B3 diabody), the C3B3 diabody (C3B3-DB), when used alone showed stronger antitumor effects than 2D7 diabody used alone (WO 2008/007755).

Because they show strong cell death-inducing activity in various human myeloma cell lines and activated lymphocytes, and demonstrate significant survival benefit in multiple myeloma model mice generated by transplanting human myeloma cell line to mice, the 2D7 diabody and the C3B3 diabody are presently under development as therapeutic agents for myeloma (WO 2004/033499; WO 2005/056603; WO 2005/100560; WO 2006/123724; WO 2008/007755; Kimura, et al., Biochem Biophys Res Commun., 325: 1201-1209 (2004)). Further advances in treatments utilizing cell death induction involving HLA class I are expected to lead to the development of highly effective pharmaceuticals for myeloma and the like.

### [Prior-art Documents]

### [Patent Documents]

[Patent Document 1] WO 2004/033499
[Patent Document 2] WO 2005/056603
[Patent Document 3] WO 2005/100560
[Patent Document 4] WO 2006/123724
[Patent Document 5] WO 2008/007755
[Non-Patent Documents]
[Non-Patent Document 1] Fayen et al., Int. Immunol., 10: 1347-1358 (1998)
[Non-Patent Document 2] Genestier et al., Blood, 90: 3629-3639 (1997)
[Non-Patent Document 3] Genestier et al., Blood, 90: 726-735 (1997)
[Non-Patent Document 4] Genestier et al., J. Biol. Chem., 273: 5060-5066 (1998)
[Non-Patent Document 5] Woodle et al., J. Immunol., 158: 2156-2164 (1997)
[Non-Patent Document 6] Matsuoka et al., J. Exp. Med., 181: 2007-2015 (1995)
[Non-Patent Document 7] Kimura, et al., Biochem Biophys Res Commun., 325: 1201-1209 (2004)
[Non-Patent Document 8] Oka, T., "Sankyo Seimei-kagaku-zaidan Kenkyu Hokoku-shu (Research Reports of the Sankyo Life Science Foundation)" (1998) 12: 46-56

### Summary of the Invention

### [Problems to be Solved by the Invention]

The present invention was achieved in view of the above circumstances. An objective of the present invention is to provide antibodies that recognize the same epitope recognized by an antibody that comprises a heavy chain having the amino acid sequence of SEQ ID NO: 2 and a light chain having the amino acid sequence of SEQ ID NO: 3. More specifically, an objective is to provide antibodies that recognize HLA class I and have significant cell death-inducing activity.

### [Means for Solving the Problems]

The present inventors conducted dedicated studies to achieve the above objectives. First, the present inventors constructed a large-scale expression system for HLA-A-FLAG (SEQ ID NO: 1) in which a flag tag is attached to the C terminus of the extracellular domain of HLA-A6802. Mice were immunized with a purified soluble HLA-A-FLAG (sHLA-flag) protein six times in total. Four days after the final immunization, spleen cells from the mice were fused with mouse myeloma P3X63Ag8U.1 cells by a conventional method using PEG 1500.

Then, hybridoma screening was carried out using cell aggregation-inducing activity and cell death-inducing activity as index. The monoclonal antibody F17B that has strong cell death-inducing activity was selected.

The sequences of H chain and L chain variable regions of the mouse monoclonal antibody F17B1 were determined to humanize this antibody. Then, the humanized anti-HLA class I antibody H2-G1d_L1-k0 was assessed for cell death induction. The result showed that H2-G1d_L1-k0 suppressed the cell growth of the IM9 cell line in a concentration-dependent manner. Furthermore, a deglycosylated form of the human chimeric antibody F17B1 was generated and administered into a mouse model for human myeloma. The result showed that that FI7B1 antibody can significantly prolong the survival period of the mouse model for human myeloma. Furthermore, analysis of subclasses of HLA class I bound by the F17B1 antibody revealed that the F17B1 IgG recognizes an epitope other than that of the 2D7 diabody or C3B3 diabody.

That is, the present inventors succeeded in producing novel anti-HLA class I antibodies that have cell death-inducing activity, and thus completed the present invention.

More specifically, the present invention provides the following.
[1] An antibody that recognizes the same epitope recognized by an antibody that comprises a heavy chain variable region having the amino acid sequence of SEQ ID NO: 2 and a light chain variable region having the amino acid sequence of SEQ ID NO: 3.
[2] The antibody of [1], which has cell death-inducing activity on a cell expressing HLA class I.
[3] The antibody of [1], which has cell growth-suppressing activity on a cell expressing HLA class I.
[4] The antibody of [1], which has cytotoxic activity on a cell expressing HLA class I.
[5] The antibody of any one of [1] to [4], which is a chimeric antibody, humanized antibody, or human antibody.
[6] A pharmaceutical composition comprising the antibody of any one of [1] to [5] as an active ingredient.
[7] The pharmaceutical composition of [6], which is a cell death-inducing agent.
[8] The pharmaceutical composition of [7], which induces cell death in a B or T cell.
[9] The pharmaceutical composition of [6], which is a cytotoxic agent.
[10] The pharmaceutical composition of [6], which is a cell growth-suppressing agent.
[11] The pharmaceutical composition of [6], which is an anti-cancer agent.
[12] The pharmaceutical composition of [11], wherein the cancer is blood cancer.
[13] A method of suppressing cell growth, which comprises the step of administering the antibody of any one of [1] to [5] to a subject.
[14] A method of treating cancer, which comprises the step of administering the antibody of any one of [1] to [5] to a subject.
[15] The antibody of any one of [1] to [5] for use in suppressing cell growth or treating cancer.
[16] Use of the antibody of any one of [1] to [5] in the manufacture of a cell growth-suppressing agent or an anti-cancer agent.

### Brief Description of the Drawings

Fig. 1 is a graph showing a comparison of the cytotoxic activities of the F17B1 and 2D7 antibodies.
Fig. 2 is a graph showing the concentration-dependent suppression of the growth of IM9 cells.
Fig. 3 is a graph showing the survival period of a mouse model for human myeloma after F17B1 administration.

### Mode for Carrying Out the Invention

The present invention relates to antibodies that recognize HLA class I.

In the present invention, HLA class I is not particularly limited. Any antigen can be used as long as it belongs to HLA class I, such as HLA-A, HLA-B, or HLA-C. In the present invention, preferable HLA class I includes, for example, HLA-A, HLA-B, and HLA-C, and particularly preferable HLA class I includes, for example, HLA-A.

The antibodies of the present invention that recognize HLA-A may be any as long as they recognize HLA-A. The antibodies may be, for example, those that specifically recognize HLA-A, or those that recognize other antigens (for example, other HLA class I antigens such as HLA-B and HLA-C) in addition to HLA-A.

More specifically, the present invention provides antibodies that recognize the same epitope recognized by an antibody of any one of (a) to (d) below:
(a) an antibody that comprises a heavy chain comprising CDR1 having the amino acid sequence of SEQ ID NO: 20, CDR2 having the amino acid sequence of SEQ ID NO: 21, and CDR3 having the amino acid sequence of SEQ ID NO: 22, and a light chain comprising CDR1 having the amino acid sequence of SEQ ID NO: 23, CDR2 having the amino acid sequence af SEQ ID NO: 24, and CDR3 having the amino acid sequence of SEQ ID NO: 25;
(b) an antibody that comprises a heavy chain variable region having the amino acid sequence of SEQ ID NO: 2 (F17 heavy chain variable region) and a light chain variable region having the amino acid sequence of SEQ ID NO: 3 (F17 light chain variable region);
(c) an antibody that comprises a heavy chain variable region having the amino acid sequence of SEQ ID NO: 4 (humanized heavy chain variable region) and a light chain variable region having the amino acid sequence of SEQ ID NO: 5 (humanized light chain variable region);
(d) an antibody that comprises a heavy chain having the amino acid sequence of SEQ ID NO: 8 (humanized heavy chain) and a light chain having the amino acid sequence of SEQ ID NO: 9 (humanized light chain); and
(e) an antibody comprising the amino acids encoded by the nucleotide sequence of SEQ ID NO: 26.

The origin of the antibodies of the present invention is not particularly limited; however, the antibodies are preferably derived from mammals, and more preferably derived from mouse or human. The antibodies of the present invention are not particularly limited as long as they recognize the same epitope recognized by an antibody of any one of (a) to (d) described above. However, it is preferred that the antibodies specifically recognize the epitope.

The antibodies used in the present invention can be obtained as polyclonal or monoclonal antibodies using known means. Mammal-derived monoclonal antibodies are particularly preferable antibodies used in the present invention. Mammal-derived monoclonal antibodies include antibodies produced by hybridomas, and antibodies produced by hosts transformed with an expression vector carrying an antibody gene using genetic engineering methods.

Basically, antibody-producing hybridomas can be prepared as follows, using conventional techniques. For example, HLA-A is used as a sensitizing antigen to perform immunizations according to conventional immunization methods. The obtained immunocytes are then fused with well-known parent cells according to conventional cell fusion methods. Monoclonal antibody-producing cells are then screened by ordinary screening methods. Antigens can be prepared by known methods, such as a method using baculoviruses (WO 98/46777 and such). Alternatively, the genetic sequence of HLA-A is inserted into a known expression vector to transform it into suitable host cells, after which the HLA-A molecule of interest is purified by known methods from the host cells or from its culture supernatant. The resulting purified HLA-A protein can then be used as the sensitizing antigen. It is also possible to use a fusion protein, composed of HLA-A molecules fused with other proteins, as the sensitizing antigen. Furthermore, HLA-B and HLA-C can also be used as the sensitizing antigens.

Mammals that are immunized with a sensitizing antigen are not particularly limited, though it is preferable to take into consideration compatibility with the parent cells used for cell fusion. Thus, rodents such as mice, rats, or hamsters are generally selected.

Immunization of animals with a sensitizing antigen is performed according to known methods. For example, standard methods of delivering a sensitizing antigen to mammals involve intraperitoneal or subcutaneous injection. More specifically, an appropriate amount of sensitizing antigen may be diluted and suspended with PBS (phosphate-buffered saline), physiological saline, or such. If desired, this may be mixed with an appropriate amount of a standard adjuvant, such as Freund's complete adjuvant, made into an emulsion, and then preferably administered to mammals several times every 4 to 21 days. An appropriate carrier may also be used during immunization with sensitizing antigens.

After such an immunization, an increase in the level of desired antibodies in the serum is confirmed, immunocytes are obtained from the mammals and the immunocytes are subjected to cell fusion. Immunocytes that are preferably subjected to cell fusion are splenocytes in particular.

Regarding other cells to be fused with the aforementioned immunocytes, mammalian myeloma cells used as parent cells include various known cell lines, such as P3X63Ag8.653 (Kearney, J. F. et al. J. Immunol. (1979) 123: 1548-1550), P3X63Ag8U.1 (Current Topics in Microbiology and Immunology (1978) 81: 1-7), NS-1 (Kohler. G. and Milstein, C. Eur. J. Immunol. (1976) 6: 511-519), MPC-11 (Margulies. D. H. et al., Cell (1976) 8: 405-415), SP2/0 (Shulman, M. et al., Nature (1978) 276: 269-270), FO (de St. Groth, S. F. et al., J. Immunol. Methods (1980) 35: 1-21), S194 (Trowbridge, I. S. J. Exp. Med. (1978) 148: 313-323), and R210 (Galfre, G. et al., Nature (1979) 277: 13,1-133).

In general, the above-mentioned immunocytes and myeloma cells may be fused according to standard methods, examples of which are described by Milstein *et al.* (Kohler. G. and Milstein, C., Methods Enzymol. (1981) 73: 3-46).

More specifically, the above-mentioned cell fusion is carried out, for example, in a standard nutrient medium in the presence of a cell fusion promoting agent. For example, polyethylene glycol (PEG), Sendai virus (HVJ), or such can be used as the fusion promoting agent. If desired, adjuvants such as dimethylsulfoxide can additionally be used to increase fusion efficiency.

Regarding the proportion of immunocytes and myeloma cells used, an example of a preferred ratio of myeloma cells to immunocytes is from 1:1 to 1:10. The medium used for the aforementioned cell fusion may be, for example, RPMI 1640 medium, MEM medium, and such, which are suitable for growth of the aforementioned myeloma cell lines, or other kinds of medium commonly used for such cell culturing. Serum supplements, such as fetal calf serum (FCS), may also be used in combination.

The cell fusion is carried out by thoroughly mixing prescribed amounts of the aforementioned immunocytes and myeloma cells in the above-mentioned medium, adding to the medium a solution of PEG preheated to about 37°C generally at a concentration of 30% to 60% (w/v), wherein the PEG has an average molecular weight of approximately 1,000-6,000, for example, and mixing them to form the desired fusion cells (hybridomas). A suitable medium is then successively added. Cell fusing agents and such that are undesirable for the growth of hybridomas can be removed by repeatedly removing the supernatant by centrifugation.

The hybridomas are selected by culturing them in a common selection medium such as HAT medium (a medium containing hypoxanthine, aminopterin, and thymidine). Culturing in the HAT medium is continued for a sufficient time, usually from a few days to a few weeks, to allow death of all cells but the target hybridomas (the non-fused cells). The usual limiting dilution method is then performed to screen and clone hybridomas producing the target antibodies.

The hybridomas prepared in this manner that produce the monoclonal antibodies may be subcultured in a common medium, and then stored for a long time in liquid nitrogen.

Monoclonal antibodies may be obtained from the hybridomas using conventional techniques; for example, the hybridomas are cultured according to standard methods and the antibodies may be obtained from the culture supernatants. Alternatively, the hybridomas are administered to a compatible mammal for proliferation and then the antibodies may be obtained from the ascites fluid. The former method is suitable for obtaining highly pure antibodies, while the latter method is more suitable for mass production of antibodies.

Recombinant antibodies produced by genetic engineering techniques can be used as the monoclonal antibodies of the present invention. They can be produced by cloning an antibody gene from a hybridoma; incorporating the antibody gene into an appropriate vector, and introducing the vector into a host (see, for example, Borrebaeck, C. A. K., and Larrick, J. W., THERAPEUTIC MONOCLONAL ANTIBODIES, Published in the United Kingdom by NIACMILLAN PUBLISHERS LTD, 1990).

Specifically, mRNAs encoding the variable (V) regions of antibodies are isolated from cells producing the target antibodies, such as hybridomas, by known methods, for example, by preparing total RNAs using guanidine ultracentrifugation methods (Chirgwin, J. M. et al., Biochemistry (1979) 18: 5294-5299), AGPC methods (Chomczynski, P. et al., Anal. Biochem. (1987) 162: 156-159), or such, and then preparing mRNAs using an mRNA Purification Kit (manufactured by GE Healthcare Biosciences) or such. The mRNAs can also be prepared directly by using a QuickPrep mRNA Purification Kit (manufactured by GE Healthcare Biosciences).

The obtained mRNAs are used to synthesize cDNAs of the antibody V regions using reverse transcriptase. cDNAs may be synthesized using the AMV Reverse Transcriptase First-strand cDNA Synthesis Kit. Alternatively, cDNA may be synthesized and amplified following the 5'-RACE method (Frohman, M. A. et al., Proc. Natl. Acad. Sci. USA (1988) 85: 8998-9002; Belyavsky, A. et al., Nucleic Acids Res. (1989) 17: 2919-2932) that uses the 5'-Ampli FINDER RACE Kit (manufactured by Clontech), and PCR. A desired DNA fragment is purified from the obtained PCR products and linked to a vector DNA. From this, a recombinant vector is constructed. The recombinant vector is then used to transform *E. coli* and such, and the desired recombinant vector is prepared from a selected colony. The nucleotide sequence of the desired DNA may then be identified through known methods, such as the deoxy method.

When DNAs encoding a V region of a desired antibody are obtained, they are linked to DNAs encoding a constant region (C region) of a desired antibody, and then these are incorporated into expression vectors. Alternatively, DNAs encoding an antibody V region can be incorporated into expression vectors that include DNAs of an antibody C region.

The antibody gene constructed as described above can be expressed using known methods. When mammalian cells are used, such an antibody gene can be expressed using a DNA in which a common useful promoter, the antibody gene to be expressed, and a poly A signal positioned downstream of the gene on the 3' side are functionally linked, or using a vector carrying the DNA. An example of a promoter/enhancer is the human cytomegalovirus immediate early promoter/enhancer.

Furthermore, as a promoter/enhancer that can be used for the expression of an antibody of the present invention, viral promoters/enhancers of retroviruses, polyomaviruses, adenoviruses, simian virus 40 (SV40), or such, or mammalian cell-derived promoter/enhancers such as human elongation factor 1α (HEF1α) or such can be used.

Antibody expression can be easily carried out by following, for example, the method of Mulligan *et al.* (Mulligan, R. C. et al., Nature (1979) 277: 108-114), using the SV40 promoter/enhancer, or the method of Mizushima *et al.* (Mizushima, S. and Nagata, S. Nucleic Acids Res. (1990) 18: 5322), using the HEF1α promoter/enhancer.

In the case of *E*. *coli,* the antibody can be expressed by an operably linked common useful promoter, a signal sequence for antibody secretion, and an antibody gene to be expressed. Examples of promoters include a lacZ promoter and an araB promoter. A lacZ promoter can be used according to the method of Ward *et al.* (Ward, E. S. et al., Nature (1989) 341: 544-546; Ward, E. S. et al., FASEB J. (1992) 6: 2422-2427) and an araB promoter can be used according to the method of Better *et al.* (Better, M. et al., Science (2988) 240: 1041-I043).

When antibody production is carried out in the periplasm of *E. coli,* the pelB signal sequence (Lei, S. P. et al. J. Bacteriol. (1987) 169: 4379-4383) may be used as a signal sequence for antibody secretion. After antibodies produced in the periplasm are separated, the antibody structure is appropriately refolded and then used (see for example WO 96/30394).

The replication origin may derive from SV40, polyoma viruses, adenoviruses, bovine papilloma viruses (BPV), and such. Furthermore, to increase the gene copy number in the host cells, the expression vector may contain, as a selection marker, an aminoglycoside phosphotransferase (APH) gene, thymidine kinase (TK) gene, *E. coli* xanthine guanine phosphoribosyl transferase (Ecogpt) gene, dihydrofolate reductase (dhfr) gene, and such.

Any production system may be used to produce the antibodies of the present invention. *In vitro* and *in vivo* production systems are available for antibody production systems. Production systems that use eukaryotic cells or prokaryotic cells are examples of *in vitro* production systems.

Production systems that use animal cells, plant cells, or fungal cells are available when using eukaryotic cells. Known animal cells include (1) mammalian cells, for example, CHO, COS, myeloma, baby hamster kidney (BHK), HeLa, Vero, and such, (2) amphibian cells such as *Xenopus laevis* oocytes, and (3) insect cells such as sf9, sf21, Tn5, and such. Known plant cells include *Nicotiana tabacum*-derived cells and these cells may be cultured as calluses. Known fungal cells include yeast, for example, the genus Saccharomyces, such as *Saccharomyces eerevisiae;* and filamentous fungi, for example, the genus Aspergillus such as *Aspergillus niger.*

Production systems that use bacterial cells are available when using prokaryotic cells. Examples of bacterial cells include *E. coli* and *Bacillus subtilis.*

Antibodies can be obtained by introducing the antibody gene of interest into these cells by transformation, then culturing these transformants *in vitro.* Transformants can be cultured using known methods. For example, DMEM, MEM, RPMI 1640, or IMDM may be used as a culture medium, and this may be used with serum supplements such as fetal calf serum (FCS). Furthermore, antibodies may be produced *in vivo* by transferring the antibody gene-introduced cells into the peritoneal cavity or such of the animals.

On the other hand, *in vivo* production systems include production systems using animals and production systems using plants. Mammals, insects and the like are used for production systems using animals.

Mammals such as goat, pig, sheep, mice, and cattle may be used (Vicki Glaser, SPECTRUM Biotechnology Applications, 1993). Alternatively, insects such as silkworms may be used. Tobacco, for example, can be used when using plants. Antibody genes are introduced into such animals or plants, upon which the antibodies are produced in the body of the animals or plants and then recovered. For example, an antibody gene is prepared as a fusion gene by inserting the antibody gene into a gene encoding a protein that is specifically produced in milk, such as the goat β-casein gene. DNA fragments containing the fusion gene to which the antibody gene has been inserted are then injected into goat embryos, which are then introduced into female goats. The desired antibody can then be obtained from milk produced by the transgenic goats, which are born from the goats that received the embryos, or from their offspring. Hormones may be suitably administered to the transgenic goats to increase the production of milk containing the antibody of interest (Ebert, K.M. et al., Bio/Technology (1994) 12: 699-702).

When silkworm is used, baculoviruses carrying an antibody gene of interest is used to infect silkworms, whereupon the antibody of interest is obtained from their body fluids (Maeda, S. et al., Nature (1985) 315: 592-594). When tobacco is used, an antibody gene of interest is inserted into a plant expression vector, for example, pMON 530, and the vector may then be introduced into a bacterium, such as *Agrobacterium tumefaciens.* The bacteria are then used to infect tobacco, such as *Nicotiana tabacum,* whereupon the desired antibodies are obtained from the leaves of this tobacco (Julian K.-C. Ma et al., Eur. J. Immunol. (1994) 24: 131-138).

When producing an antibody using an *in vitro* or *in vivo* production system as described above, a DNA encoding the heavy chain (H chain) or the light chain (L chain) of the antibody can be each separately incorporated into a expression vector to simultaneously transform the host cell, or alternatively, a DNA encoding the H chain and the L chain can be incorporated into a single expression vector to transform the host cell (see International Patent Application No. WO 94/11523).

In the present invention, artificially modified genetically-recombinant antibodies, such as chimeric and humanized antibodies, may be used to reduce heterologous antigenicity against humans. These modified antibodies can be produced using known methods. A chimeric antibody is an antibody composed of the heavy and light chains variable regions of an antibody from a non-human mammal, such as a mouse, and the heavy and light chains constant regions of a human antibody. The chimeric antibody can be produced by linking a DNA encoding a mouse antibody variable region with a DNA encoding a human antibody constant region, incorporating this into an expression vector, and then introducing the vector into a host.

The chimeric antibody can be produced by linking a DNA encoding the antibody V regions obtained as described above with a DNA encoding the C regions of the human antibody, incorporating this into an expression vector, and then introducing the vector into a host (see European Patent Application No. EP 125023 and International Patent Application No. WO 92/19759). Chimeric antibodies useful for the present invention can be obtained using this known method.

Humanized antibodies are also referred to as "reshaped human antibodies". Such humanized antibodies are obtained by grafting the complementarity determining region (CDR) of an antibody derived from a non-human mammal, for example, a mouse, to the CDR of a human antibody. Such general gene recombination procedures are also known.

For example, a DNA sequence designed to link a murine antibody CDR to the framework region (FR) of a human antibody is synthesized by PCR, using several oligonucleotides produced to contain overlapping portions in the terminal regions. The obtained DNA is linked to a DNA encoding a human antibody constant region, and incorporated into an expression vector. The antibody is produced by introducing this vector into a host (see European Patent Application No. EP 239400, and International Patent Application No. WO 96/02576).

The human antibody FR to be linked *via* CDR is selected such that the CDR forms a favorable antigen-binding site. In order for the CDR of the reshaped human antibody to form a suitable antigen-binding site, the amino acids in the framework region of the antibody variable region may be substituted as necessary (Sato, K. et al., Cancer Res. (1993) 53: 851-856).

Human antibody constant regions are used for the chimeric antibodies and humanized antibodies. Examples of the human antibody heavy chain constant regions to be used include IgG1 constant regions, IgG2 constant regions, IgG3 constant regions, and IgG4 constant regions. Examples of the human antibody light chain constant regions to be used includes chain constant regions and κ chain constant regions. Furthermore, the human antibody constant regions may be modified by amino acid substitution, deletion, or the like, to improve antibody stability, stability of antibody production, etc.

A chimeric antibody includes the variable region of an antibody derived from a non-human mammal and the C-region derived from a human antibody. A humanized antibody is composed of the CDR of an antibody derived from a non-human mammal and a framework region and C region derived from a human antibody. Since the antigenicity of both these antibodies is low in human body, they are suitable as antibodies used in pharmaceutical agents.

Methods for obtaining human antibodies are also known. For example, human lymphocytes can be sensitized *in vitro* with a desired antigen, or with cells expressing a desired antigen, and the sensitized lymphocytes can be fused with human myeloma cells, such as U266, to obtain the desired human antibody with antigen-binding activity (see Japanese Patent Application Kokoku Publication No. (JP-B) H01-59878 (examined, approved Japanese patent application published for opposition)). Further, a desired human antibody can be obtained by using a desired antigen to immunize transgenic animals that have a full repertoire of human antibody genes (see International Patent Application No. WO 93/12227, WO 92/03918, WO 94/02602, WO 94/25585, WO 96/34096, and WO 96/33735). Additional known techniques for obtaining human antibodies include panning using a human antibody library. For example, variable regions of human antibodies can be expressed as single-chain antibodies (scFvs) on the surface of phages using phage display methods, from which phages that bind to antigens can be selected. DNAS sequences encoding the variable regions of human antibodies that bind to the antigens can be determined by analyzing the genes of the selected phages. By revealing the DNA sequences of the scFvs that bind to the antigens, appropriate expression vectors carrying the sequences can be produced to yield human antibodies. These methods are already known, and the following publications can be referred to: WO 92/01047, WO 92/20791, WO 93/06213, WO 93/11236, WO 93/19172, WO 95/01438, and WO 95/15388.

Antibodies used in the present invention may be low-molecular-weight antibodies. In the present invention, low-molecular-weight antibodies (minibodies) include antibody fragments derived from a parent antibody, i.e. a whole antibody (for example, whole IgG), in which part of the whole antibody is missing; the antibody fragment is not particularly limited so long as it has antigen-binding ability. Antibody fragments of the present invention are not particularly limited so long as they are part of a whole antibody. However, fragments containing heavy chain variable regions (VH) or light chain variable regions (VL) are preferred, and fragments containing both VH and VL are particularly preferred. Specific examples of antibody fragments include Fab, Fab', F(ab')₂, Fv, scFv (single chain Fv), sc(Fv)₂ and such, but are preferably diabodies (Huston, J. S. et al., Proc. Natl. Acad. Sci. USA (1988) 85: 5879-5883; Plickthun "The Pharmacology of Monoclonal Antibodies" Vol.113, Resenburg and Moore ed., Springer Verlag, New York, pp.269-315, (1994)). Such antibody fragments can be obtained by treating an antibody with enzymes such as papain or pepsin to produce antibody fragments, or by constructing genes encoding such antibody fragments, introducing them into an expression vector, and then expressing this in an appropriate host cell (see for example, Co, M. S. et al., J. Immunol. (1994) 152: 2968-2976; Better, M. and Horwitz, A. H., Methods Enzymol. (1989) 178: 476-496; Pluckthun, A. and Skerra, A., Methods Enzymol. (1989) 178: 497-515; Lamoyi, E., Methods Enzymol. (1986) 121: 652-663; Rousseaux, J. et al, Methods Enzymol. (1986) 121: 663-669; Bird, R. E. and Walker, B. W., Trends Biotechnol. (1991) 9: 132-137).

The molecular weight of the low-molecular-weight antibody of the present invention is preferably smaller than that of the whole antibody; however, multimers such as dimers, trimers, and tetramers may be formed, such that the molecular weight can be larger than the molecular weight of the whole antibody.

Examples of low-molecular-weight antibodies of the present invention include antibodies composed of two or more VH and two or more VL of an antibody, and these variable regions are linked directly or indirectly through linkers or such. The linkages may be covalent bonds, non-covalent bonds, or both covalent and non-covalent bonds. A more preferred low-molecular-weight antibody is an antibody composed of two or more VH-VL pairs formed by linking VH and VL with a non-covalent bond. In this case, a low-molecular-weight antibody having a shorter distance between one VH-VL pair and the other VH-VL pair than the distance in the whole antibody is preferred.

In the present invention, seFv is obtained by ligating an antibody H chain V region with an antibody L chain V region. In this scFv, the H chain V region and L chain V region are ligated *via* a linker, preferably *via* a peptide linker (Huston, J. S. et al., Proc. Natl. Acad. Sci. USA (1988) 85: 5879-5883). The H chain V region and L chain V region in scFv may be derived from any of the antibodies described herein. For example, any single-chain peptides composed of 12 to 19 amino acid residues may be used as a peptide linker for ligating the V regions.

DNAs encoding scFv can be obtained by using as a template, DNAs encoding the antibody H chain or H chain V region and the antibody L chain or L chain V region mentioned above, and, from among those sequences, amplifying a DNA portion that encodes the desired amino acid sequence by PCR using a primer pair that defines its two ends; and then carrying out a subsequent amplification using a combination of a DNA encoding the peptide linker portion, and the primer pair that defines both ends of the linker DNA to be ligated to the H chain and the L chain, respectively.

Once a DNA encoding an scFv is constructed, an expression vector containing the DNA, and a host transformer with the expression vector can be obtained according to conventional methods. Furthermore, scFvs can be obtained using these hosts according to conventional methods.

These antibody fragments can be produced in hosts by obtaining their genes and expressing them in a manner similar to that described above. These antibody fragments are included as "antibodies" of the present invention.

Low-molecular-weight antibodies that are particularly preferred in the present invention are diabodies. Diabodies are dimers formed by linking two fragments (such as scFvs; hereinafter referred to as diabody-constituting fragments), in which a variable region is linked to another variable region *via* a linker or such. Ordinarily, diabodies composed of two VLs and two VHs (P. Holliger et al., Proc. Natl. Acad. Sci. USA (1993) 90: 6444-6448; EP 404097; WO 93/11161; Johnson et al., Method in Enzymology (1991) 203: 88-98; Holliger et al., Protein Engineering (1996) 9: 299-305; Perisic et al., Structure (1994) 2: 1217-1226; John et al., Protein Engineering (1999) 12 (7): 597-604; Holliger et al, . Proc. Natl. Acad. Sci. USA (1993) 90: 6444-6448; Atwell et al., Mol. Immunol. (1996) 33: 1301-1312). Bonds between diabody-constituting fragments may be non-covalent or covalent bonds, but are preferably non-covalent bonds.

Alternatively, diabody-constituting fragments may be linked to each other by a linker and such to form a single-chain diabody (sc diabody). In such cases, linking diabody-constituting fragments using a long linker of about 20 amino acids allows the diabody-constituting fragments on the same chain to form a dimer with each other *via* non-covalent bonds.

Diabody-constituting fragments include those with linked VL-VH, VL-VL, and VH-VH, more preferably those with linked VH-VL. In diabody-constituting fragments, the linker used to link a variable region to a variable region is not particularly limited, but is preferably short enough to prevent non-covalent bonding between variable regions in the same fragment. The length of such a linker can be suitably determined by those skilled in the art, and is ordinarily 2 to 14 amino acids, preferably 3 to 9 amino acids, and more preferably 4 to 6 amino acids. In this case, linkers between VL and VH encoded on a same fragment are short, and thus VL and VH on a same strand do not form a non-covalent bond; therefore a single-chain V region fragment will not be formed. Rather, a fragment forms a dimer with another fragment *via* non-covalent bonding. Furthermore, according to the same principle in diabody construction, three or more diabody-constituting fragments may be linked to form multimeric antibodies such as trimers and tetramers. Examples of diabodies include the above-mentioned C3B3 diabody (WO 2008/007755), 2D7 diabody (Kimura, et al., Biochem Biophys Res Commun., 325: 1201-1209 (2004)), and such, but are not limited thereto.

Whether an antibody recognizes the same epitope recognized by an antibody of any one of (a) to (d) above can be confirmed by the competition between the two antibodies against the epitope. Competition between the antibodies can be evaluated by competitive binding assays using means such as ELISA, fluorescence energy transfer method (FRET), and fluorometric microvolume assay technology (FMAT^{®}).

The amount of the antibody of any one of (a) to (d) above that is bound to an antigen indirectly correlates with the binding ability of candidate competitor antibodies (test antibodies) that competitively bind to the same epitope. In other words, as the amount of or the affinity of test antibodies against the same epitope increases, the amount of the antibody of any one of (a) to (d) above that is bound to the antigen decreases, and the amount of test antibodies bound to the antigen increases. Specifically, the antibody of any one of (a) to (d) above that is appropriately labeled and an antibody to be evaluated are simultaneously added to an antigen, and thus the antibody of any one of (a) to (d) above that is bound to the antigen is detected using the label. The amount of the antibody of any one of (a) to (d) above that is bound to the antigen can be easily determined by labeling the antibody beforehand. This label is not particularly limited, and the labeling method is selected according to the assay technique used. The labeling method includes fluorescent labeling, radiolabeling, enzymatic labeling, and such.

For example, the antibody of any one of (a) to (d) above that is fluorescently labeled and the antibody of any one of (a) to (d) above that is unlabeled or a test antibody are simultaneously added to animal cells expressing HLA-A, and the labeled antibody is detected by fluorometric microvolume assay technology.

IC₅₀ is the concentration in which the amount of the antibody of any one of (a) to (d) above that is labeled and bound to the epitope is reduced to 50% by the binding of the antibody of any one of (a) to (d) above that is unlabeled. Herein, an antibody that recognizes the same epitope is an antibody that can reduce the amount of the antibody of any one of (a) to (d) above that is labeled and bound to the epitope to at least 50% when the concentration of the test antibody is usually 100 times, preferably 50 times, more preferably 30 times, still more preferably ten times higher than the IC₅₀ of the unlabeled antibody.

When two antibodies are assayed for competition between them, the constant regions of the antibodies are not particularly limited; however, it is preferred that the constant regions are of the same subtype.

In the present invention, HLA class I used to assess whether antibodies recognize the same epitope is not particularly limited. It is possible to use any antigen that belongs to HLA class I such as HLA-A, HLA-B, or HLA-C. Preferable HLA class I include, for example, HLA-A, HLA-B, and HLA-C, and particularly preferable HLA class I includes, for example, HLA-A.

Antibodies that bind to an epitope to which an antibody of any one of (a) to (d) described above binds are useful as pharmaceutical agents because they have very strong cell death-inducing activity.

When the antibody of (a) described above, which comprises a heavy chain comprising CDR1 having the amino acid sequence of SEQ ID NO: 20, CDR2 having the amino acid sequence of SEQ ID NO: 21, and CDR3 having the amino acid sequence of SEQ ID NO: 22, and a light chain comprising CDR1 having the amino acid sequence of SEQ ID NO: 23, CDR2 having the amino acid sequence of SEQ ID NO: 24, and CDR3 having the amino acid sequence of SEQ ID NO: 25, has FR and/or constant regions, the regions are not particularly limited, and the antibody may comprise any FR and/or constant region.

When the antibody of (b) described above, which comprises a heavy chain variable region having the amino acid sequence of SEQ ID NO: 2 (F1.7 heavy chain variable region) and a light chain variable region having the amino acid sequence of SEQ ID NO: 3 (F17 light chain variable region), or the antibody of (c) described above, which comprises a heavy chain variable region having the amino acid sequence of SEQ ID NO: 4 (humanized heavy chain variable region) and a light chain variable region having the amino acid sequence of SEQ ID NO: 5 (humanized light chain variable region), has a constant region, the region is not particularly limited, and the antibody may comprise any constant region.

Preferable heavy chain constant regions include, for example, human IgG1 constant regions, human IgG2 constant regions, human IgG3 constant regions, human IgG4 constant regions, and modified constant regions comprising one or more amino acid substitutions, deletions, additions and/or insertions in the above constant regions. Such modified constant regions include, for example, a constant region resulting from a combination of human IgG1 constant region and human IgG2 constant region (for example, a constant region whose hinge region is derived from human IgG2 and the remaining portion is derived from human IgG1).

Preferably, the antibodies of the present invention are antibodies that have a biological action on cells when the antibodies bind to HLA class I expressed on the cells. Examples of such biological actions include cytotoxic actions, anti-tumor actions, and such, although they are not limited thereto. More specific examples include cell death-inducing actions, apoptosis-inducing actions, cell growth-suppressing actions, cell differentiation-suppressing actions, cell division-suppressing actions, cell growth-inducing actions, cell differentiation-inducing actions, cell division-inducing actions, and cell cycle-regulating actions. Cell death-inducing actions and cell growth-suppressing actions are preferred.

Target cells of the above-mentioned actions, such as cell death-inducing actions and cell growth-suppressing actions, are not particularly limited, although immune cells, hematopoietic cells, and non-adherent cells are preferred. Specific examples of hematopoietic cells include lymphocytes (B cells, T cells), NK cells, NKT cells, neutrophils, eosinophils, basophils, monocytes (preferably activated peripheral blood mononuclear cells (PBMC)), and myeloma cells, while lymphocytes (B cells, T cells), and myeloma cells are preferred, and T cells or B cells (in particular, activated B cells or T cells) are most preferable. The phrase "non-adherent cells" refer to cells that, when cultured, grow in a non-adherent state without adhering to the surface of culturing vessels made of glass, plastic or the like. On the other hand, the phrase "adherent cells" refer to cells that, when cultured, adhere to the surface of culturing vessels made of glass, plastic or the like.

In general, it is possible to crosslink the anti-HLA class I antibodies with an anti-IgG antibody or the like to enhance their biological action such as cell death-inducing activity. Thus, the antibodies that have a biological action upon binding to HLA-A-expressing cells may be antibodies that have the biological action when they are crosslinked with another antibody such as an anti-IgG antibody, or antibodies that have the biological action without crosslinkage with another antibody. Such crosslinkage can be achieved by methods known to those skilled in the art.

The antibodies of the present invention are useful, in particular, as pharmaceutical agents. Thus, the present invention provides pharmaceutical compositions comprising an antibody of the present invention. The present invention also provides therapeutic pharmaceutical compositions comprising an antibody of the present invention.

The antibodies of the present invention are useful as cell death-inducing agents to induce cell death in cells such as cancer cells. Thus, the present invention provides cell death-inducing agents comprising an antibody of the present invention.

The antibodies of the present invention are also useful as anticancer agents. Thus, the present invention provides anticancer agents comprising an antibody of the present invention.

When the disease targeted by a pharmaceutical composition of the present invention is cancer, the type of cancer is not particularly limited, and may be any such as myeloma, blood cancer (blood tumor), liver cancer, lung cancer, colon cancer, breast cancer, prostatic cancer, leukemia, lymphoma, pancreatic cancer, and bile duct cancer. The cancer may be primary or secondary. Preferable cancers include myeloma and blood cancer (blood tumor). Specifically, blood cancer (blood tumor) includes, for example, leukemia, myelodysplastic syndrome, malignant lymphoma, Burkitt's lymphoma, chronic myelocytic leukemia, acute myelocytic leukemia, plasma cell abnormalities (myeloma, multiple myeloma, and macroglobulinaemia), myeloproliferative disorders (polycythemia rubra vera, essential thrombocythemia, idiopathic myelofibrosis), adult T cell leukemia (ATL), etc. In a preferred embodiment, cancers targeted by anticancer agents of the present invention include cancers in which the expression of HLA is enhanced as compared to normal cells. Furthermore, the antibodies of the present invention can also be used as therapeutic agents for autoimmune diseases or as agents for suppressing rejections in transplantation.

The pharmaceutical compositions of the present invention may be used together with other agents for maintenance therapy.

Subjects to which pharmaceutical compositions of the present invention are administered are mammals. The mammals are preferably humans.

The pharmaceutical compositions of the present invention can be administered in the form of a pharmaceutical, and can be administered orally or parenterally and systemically or topically. For example, intravenous injection such as drip infusion, intramuscular injection, intraperitoneal injection, subcutaneous injection, suppository, colonic infusion, oral enteric coating agent, trancutaneous administration, or transpulmonary administration may be selected, and a suitable administration method can be selected according to the age and symptoms of the patient. The effective dose can be selected from the range of 0.01 mg to 100 mg per kg body weight in each administration. Alternatively, the dosage can be selected from 1-1,000 mg per patient, or preferably 5-50 mg per patient. For example, preferred dose and method of administration refer to an effective dose, which is an amount that causes free antibodies to be present in the blood, and specific examples include administration methods such as administering 0.5 mg to 40 mg per month (four weeks) per kg body weight, which is preferably one mg to 20 mg in one to several doses, for example, by methods including intravenous injection such as drip infusion or subcutaneous injection following an administration schedule of twice/week, once/week, once/two weeks, once/four weeks, or such. The administration schedule can be adjusted by extending the administration interval from twice/week or once/week to once/two weeks, once/three weeks, or once/four weeks by observing post-administration condition and changes in blood test values.

Pharmaceutically acceptable carriers, such as preservatives and stabilizers, can be added to the pharmaceutical compositions of the present invention. The term "pharmaceutically acceptable carrier" refers to a carrier that in itself may be a material that has or does not have the above-described cytotoxic activity; the carrier is a material that can be administered together with the pharmaceutical composition of the present invention. It may further be a material that does not have the cytotoxic activity, but has a synergistic or additive stabilizing effect when used in combination with an anti-HLA class I antibody.

Examples of pharmaceutically acceptable materials include sterilized water, physiological saline, stabilizers, excipients, buffers, preservatives, surfactants, chelating agents (for example, EDTA), and binders and the like.

In the present invention, examples of surfactants include non-ionic surfactants. Typical examples include, sorbitan fatty acid esters such as sorbitan monocaprilate, sorbitan monolaurate, or sorbitan monopalmitate; glycerol fatty acid esters such as glycerol monocaprilate, glycerol monomyristate, or glycerol monostearate; polyglycerol esters of fatty acids such as decaglyceryl monostearate, decaglyceryl distearate, or decaglyceryl monolinoleate; polyoxyethylene sorbitan fatty acid esters such as polyoxyethylene sorbitan monolaurate, polyoxyethylene sorbitan monooleate, polyoxyethylene sorbitan monostearate, polyoxyethylene sorbitan monopalmitate, polyoxyethylene sorbitan trioleate, or polyoxyethylene sorbitan tristearate; polyoxyethylene sorbitol fatty acid esters such as polyoxyethylene sorbitol tetrastearate, polyoxyethylene sorbitol tetraoleate; polyoxyethylene glycerol fatty acid esters such as polyoxyethylene glyceryl monostearate; polyethylene glycol fatty acid esters such as polyethylene glycol distearate; polyoxyethylene alkyl ether such as polyoxyethylene lauyl ether; polyoxyethylene polyoxypropylene alkyl ether such as polyoxyethylene polyoxypropylene glycol, polyoxyethylene polyoxypropylene propylether, or polyoxyethylene polyoxypropylene cetyl ether; polyoxyethylene alkylphenyl ether such as polyoxyethylene nonylphenyl ether; polyoxyethylene hardened castor oils such as polyoxyethylene castor oil, or polyoxyethylene hardened castor oil (polyoxyethylene hydrogenated castor oil); polyoxyethylene beeswax derivatives such as polyoxyethylene sorbitol beeswax; polyoxyethylene lanolin derivatives such as polyoxyethylene lanolin; and polyoxyethylene fatty acid amide with HLB 6 to 18 such as polyoxyethylene stearylamide.

Anionic surfactants can also be listed as surfactants. Typical examples of anionic surfactants may include alkyl sulfate salts having an alkyl group of 10 to 18 carbon atoms, such as sodium cetyl sulfate, sodium lauryl sulfate, or sodium oleyl sulfate; polyoxyethylene alkylether sulfate salts whose average mole of ethyleneoxide added is two to four and the number of carbon atoms in the alkyl group is 10 to 18, such as sodium polyoxyethylene lauryl sulfate; alkyl sulfosuccinate ester salts of 8 to 18 carbon atoms in the alkyl group, such as sodium lauryl sulfosuccinate ester; naturally-occurring surfactants such as lecithin or glycerol lipid phosphate; sphingophospholipids such as sphingomyelin; and sucrose fatty acid esters of 12 to 18 carbon atoms in the fatty acid.

One or a combination of two or more of these surfactants can be added to the pharmaceutical compositions of the present invention. A preferred surfactant to be used in the pharmaceutical composition of the present invention is a polyoxyethylene sorbitan fatty acid ester, such as Polysorbate 20, 40, 60, 80, or such, wherein Polysorbate 20 and 80 are particularly preferred. Polyoxyethylene polyoxypropylene glycol represented by Poloxamer (for example, Pluronic F-68®) is also preferred.

The amount of surfactant to be added differs depending on the type of surfactant used, but for Polysorbate 20 or Polysorbate 80, it is generally 0.001-100 mg/mL, preferably 0.003-50 mg/mL, and more preferably 0.005-2 mg/mL.

Examples of buffers in the present invention include such as phosphoric acid, citric acid buffer, acetic acid, malic acid, tartaric acid, succinic acid, lactic acid, calcium phosphate, gluconic acid, caprylic acid, deoxycholic acid, salicylic acid, triethanolamine, fumaric acid, other organic acids, carbonic acid buffer, Tris buffer, histidine buffer, imidazole buffer and the like.

Solution formulations can be prepared by dissolving into aqueous buffers that are known in the field of solution formulation. The buffer concentration is generally 1-500 mM, preferably 5-100 mM, and even more preferably 10-20 mM.

The pharmaceutical compositions of the present invention may further include other low-molecular-weight polypeptides, proteins such as serum albumin, gelatin, and immunoglobulin, amino acids, sugars and carbohydrates such as polysaccharides and monosaccharides, and sugar alcohols.

Examples of amino acids in the present invention include basic amino acids such as arginine, lysine, histidine, and ornithine, and inorganic salts of these amino acids (preferably in the form of chloride salts or phosphate salts, or more specifically amino-acid phosphates). When using free amino acids, the pH is adjusted to a preferred value by adding a suitable physiologically acceptable buffer, such as inorganic acids, particularly hydrochloric acid, phosphoric acid, sulfuric acid, acetic acid, formic acid, or a salt thereof. In such cases, the use of a phosphoric acid salt is particularly useful because a particularly stable freeze-dried product can be obtained. It is particularly advantageous when the preparation does not substantially contain an organic acid such as malic acid, tartaric acid, citric acid, succinic acid, or fumaric acid, or when a corresponding anion (maleate ion, tartarate, ion, citrate ion, succinate ion, fumarate ion, or such) is not present. Preferred amino acids are arginine, lysine, histidine, or ornithine. Furthermore, acidic amino acids such as glutamic acid and aspartic acid, and salts thereof (preferably sodium salts); neutral amino acids such as isoleucine, leucine, glycine, serine, threonine, valine, methionine, cysteine, or alanine; or aromatic amino acids such as phenylalanine, tyrosine, tryptophan, or its derivative, N-acetyltryptophan can be used.

Examples of sugars and carbohydrates, such as polysaccharides and monosaccharides, in the present invention include dextran, glucose, fructose, lactose, xylose, mannose, maltose, sucrose, trehalose, and raffinose.

Examples of sugar alcohols in the present invention include mannitol, sorbitol, inositol, and such.

When the pharmaceutical composition of the present invention is used as an aqueous solution used for injection, the solution can be mixed with, for example, physiological saline and isotonic solutions that include glucose or other adjunctive agents such as D-sorbitol, D-mannose, D-mannitol, and sodium chloride. The solution may also be combined with appropriate solubilizing agents, such as alcohol (for example, ethanol), polyalcohol (for example, propylene glycol or PEG), or non-ionic surfactant (for example, polysorbate 80 or HCO-50). If desired, diluents, solubilizing agents, pH-adjusting agents, soothing agents, sulfur-containing reducing agents, and antioxidants may be included.

Examples of sulfur-containing reducing agents in the present invention include N-acetyl cysteine, *N*-acetyl homocysteine, thioctic acid, thiodiglycol, thioethanolamine, thioglycerol, thiosorbitol, thioglycolic acid, and salts thereof, sodium thiosulfate, glutathione, and compounds carrying a sulfhydryl group such as thioalkanoic acid of one to seven carbon atoms.

Examples of antioxidants in the present invention include erythorbic acid, dibutylhydroxytoluene, butylhydroxyanisole, α-tocopherol, tocopherol acetate, L-ascorbic acid and salts thereof, L-ascorbyl palmitate, L-ascorbyl stearate, sodium bisulfite, sodium sulfite, triamyl gallate, propyl gallate, and chelating agents such as ethylenediamine tetraacetic acid disodium (EDTA), sodium pyrophosphate, and sodium metaphosphate.

If necessary, the pharmaceutical agents can be contained within microcapsules (microcapsules made of hydroxymethylcellulose, gelatin, poly[methyl methacrylate], or such), or made into colloidal drug delivery systems (such as liposomes, albumin microspheres, microemulsion, nanoparticles, and nanocapsules) (see for example, "Remington's Pharmaceutical Science 16th edition", Oslo Ed., 1980). Methods for preparing the pharmaceutical agents as controlled-release pharmaceutical agents are also well known, and such methods may be applied to the present invention (Langer et al., J. Biomed. Mater. Res. (1981) 15: 167-277; Langer, Chem. Tech. (1982) 12: 98-105; U.S. Patent No. 3,773,919; European Patent (EP) Patent Application No. 58,481; Sidman et al., Biopolymers (1983) 22: 547-556; EP 133,988).

Pharmaceutically acceptable carriers used are suitably selected from those mentioned above or combinations thereof according to the dosage form, but are not limited thereto.

All prior art documents cited in this specification are incorporated herein by reference.

### Examples

Hereinbelow, the present invention is specifically described with reference to the Examples, but it is not to be construed as being limited thereto.

### [Example 1] Preparation of mouse anti-HLA class I antibodies

### Hybridoma preparation

### 1.1. Preparation of soluble HLA-A-FLAG (sHLA-A-flag) as immunogen

### 1. 1. 1. Construction of an sHLA-A-flag expression vector and establishment of an sHLA-A-flag-producing CHO line

sHLA-A-flag (SEQ ID NO: 1, a FLAG tag is located at positions 307 to 314), in which the flag tag is attached to the C terminus of the extracellular domain of HLA-A6802 (GenBank No. AM235885), was inserted into an animal cell expression vector. The nucleotide sequence was determined by a DNA sequencer, ABI PRISM 3700 DNA Sequencer (Applied Biosystems) using a BigDye Terminator Cycle Sequencing Kit (Applied Biosystems) according to the method described in the appended instruction manual. The constructed expression vector was introduced into CHO cells to establish an sHLA-A-flag-producing CHO line.

### 1.1.2. Purification of sHLA-A-flag

Using Q-Sepharose FF (GE Healthcare), ANTI-FLAG M2 Affinity Gel (SIGMA), and HiLoad 16/60 Superdex 200 pg (GE Healthcare), the sHLA-A-flag protein was purified from the culture supernatant of the CHO cells prepared as described in 1.1.1. The eluted gel filtration fraction was concentrated using Centriprep YM-10 (Millipore), and the protein concentration was calculated using a Dc Protein Assay Kit (BIO-RAD) and bovine gamma globulin as a standard.

### 1.2. Preparation of hybridomas producing mouse anti-HLA class I antibodies

### 1.2.1. Preparation of hybridomas using mice immunized with sHLA-A-flag

Four-week-old male MRL/lpr mice (Charles River Japan, Inc.) were immunized. In the primary immunization, an emulsion was prepared by mixing complete adjuvant H37Ra (DIFCO #231131) with purified sHLA-A-flag protein (100 µg/head) based on the number of mice to be used. Each mouse was immunized with 100 µl of the emulsion by subcutaneous injection. In the second and subsequent immunizations, an emulsion was prepared using incomplete adjuvant (DIFCO #263910), and the mice were immunized at 50 µg/head. Immunization was carried out at one-week intervals. After the serum antibody titer was confirmed to be elevated, the final immunization (sixth immunization) was performed by preparing sHLA-A-flag at 50 µg/200 µl in PBS and intravenously administering it to the mice at 200 µl/head.

Four days after the final immunization, spleen cells from the mice were fused with mouse myeloma P3X63Ag8U.1 cells (abbreviated as P3U1; ATCC CRL-1597) by a conventional cell fusion method using PEG 1500 (Roche Diagnostics). The fused cells, i.e., hybridomas, were cultured in HAT medium [RPMI 1640 + PS, 10% FCS, HAT (Sigma, H0262), 5% BM condimed H1 (Roche, #1088947)].

### 1.2.2. Hybridoma selection

About one week after the fusion, primary screening was carried out using cell aggregation-inducing activity as index. The cell aggregation-inducing activity was assessed as follows. ARH77 cells were plated at 2 x 10⁴ cells/well (40 µl) in 96-well plates, 80 µl each of the hybridoma culture supernatants was added thereto, and the cells were incubated at 37°C for four hours. Each well was observed under a microscope, and cell aggregation within the well was visually assessed. Hybridomas that showed cell aggregation were selected as positive clones. Hybridomas in the wells that were determined to be positive for the cell aggregation-inducing activity were plated at 2.5 cells/well in 96-well plates. After about ten days of culture, the hybridomas were assessed again for the cell aggregation-inducing activity. A secondary screening was performed using the cell death-inducing activity of antibody as index. ARH77 cells were plated at 1 x 10⁵ to 8 x 10⁵ cells/well in 24-well plates. Each antibody was added to the plates. The cells were incubated in 200 µl of medium at 37°C for four to five hours or more, and then transferred into microtubes. The cells were suspended in 100 to 200 µl of a propidium iodide (PI) solution (5 µg/ml in FACS buffer) and incubated at room temperature for 15 minutes. After precipitation by centrifugation, the cells were suspended in 500 µl FACS buffer. The proportion of PI-positive cells (nonviable cells) was determined by FACS (Beckman Coulter, ELITE). The cell death-inducing activity of the clones was compared. As a result, clone F17B1 that has strong cell death-inducing activity was selected.

### [Example 2] Comparison of the cytotoxic activity of the F17B1, 2D7, and C3B3 antibodies

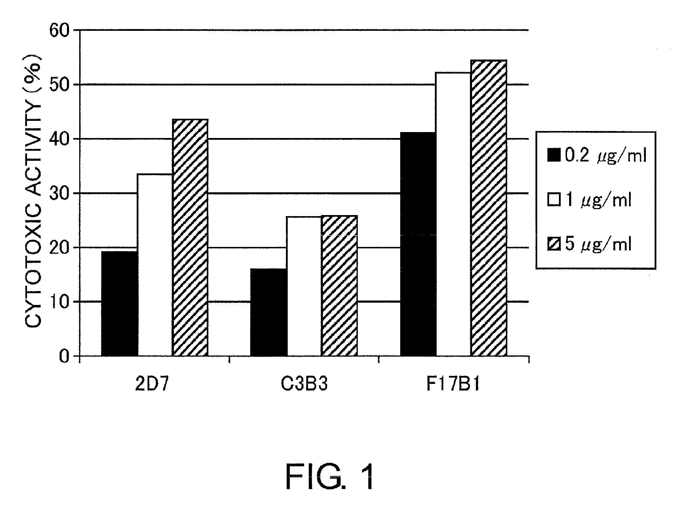

The F17B1 antibody prepared as described in Example 1, and the 2D7 and C3B3 antibodies, which recognize HLA class I, were added to ARH77 cells at 0.2, 1, or 5 µg/ml. After the addition of antibodies, the cells were incubated at 37°C for four to five hours. Then, the cells were stained with PI, and the proportion of nonviable cells was determined by FACS analysis (Fig. 1).

### [Example 3] Determination of the variable regions of the mouse anti-HLA class I antibody F17B1

Total RNA was extracted from hybridomas using RNeasy Mini Kits (QIAGEN), and cDNA was synthesized using a SMART RACE cDNA Amplification Kit (BD Biosciences). Using PCR on the prepared cDNA, antibody variable region genes were inserted into cloning vectors. The nucleotide sequence of each DNA fragment was determined by a DNA sequencer, ABI PRISM 3700 DNA Sequencer (Applied Biosystems) using a BigDye Terminator Cycle Sequencing Kit (Applied Biosystems) according to the method described in the appended instruction manual. The determined H chain and L chain variable regions of the mouse F17B1 antibody are shown in SEQ ID NOs: 2 and 3, respectively. The CDRs and FRs were determined according to Kabat numbering.

### [Example 4] Preparation of a humanized anti-HLA class I antibody

The variable region sequences of the mouse F17B antibody were compared to human germline sequences and human antibody sequences to determine the FR sequences to be used for antibody humanization. The FR sequences used are summarized in Table 1.

The humanized H chain variable region H2 (SEQ ID NO: 4) comprises For1, FR2, FR3, and FR4 shown in Table 1. The nucleotide sequence encoding H2_G1d (SEQ ID NO: 8), in which H2 thus constructed is linked with the constant region G1d (SEQ ID NO: 6), was inserted into an animal cell expression vector.

The humanized L chain variable region (SEQ ID NO: 5) comprises FR1, FR2, FR3, and FR4 shown in Table 1. FR3 comprises the following two types of germline sequences: hVk2_28 (GVPDRFSGSGSGTDFTLKISRVEAEDVGVYYC; SEQ ID NO: 18) and Vk6_21 (GVPSRFSGSGSGTDFTLTINSLEAEDAATYYC; SEQ ID NO: 19). The nucleotide sequence encoding L1_k0 (SEQ ID NO: 9), in which L1 thus constructed is linked with the constant region k0 (SEQ ID NO: 7), was inserted into a mammalian cell expression vector.

H2_Gld/L1_k0 comprising H2_G1d (SEQ ID NO: 8) as H chain and L1_k0 (SEQ ID NO: 9) as L chain was prepared using the constructed expression vectors. The antibody was expressed and purified by the methods described in Reference Example 1.

**[Table 1]**

| H2 | Reference sequence | Human FR sequence | SEQ ID NO: |
|---|---|---|---|
| FR1 | Accession No. U96288 | QVTLKESGPTLVKPTQTLTLTCAFSGFSLN | 10 |
| FR2 | Germline:hVH2_70 | VVLRQPPGKALEWLA | 11 |
| FR3 | Accession No. AY393293 | RLTISKDSSKNQVDLTMTNMDPVDTATYYCAR | 12 |
| FR4 | Germline:JH1 | VvGQGTLVTVSS | 13 |
| | | | |

| L1 | Reference sequence | Human FR sequence | SEQ ID NO: |
|---|---|---|---|
| FR1 | Germline:hVk3_11 | EIVLTQSPATLSLSPGERATLSC | 14 |
| FR2 | Germline:hVk3_11 | WYQQKPGQAPRLLIY | 15 |
| FR3 | hVk2_28+hVk6_21 | GVPDRFSGSGSGTDFTLTINSLEAEDAATYYC | 16 |
| FR4 | Germline:JK2 | FGQGTKLEIK | 17 |

### [Example 5] Cell death induction by the humanized anti-HLA class I antibody H2-Gld L1-k0

The humanized anti-HLA class I antibody H2-G1d L1-k0 was assessed for the cell death-inducing activity using the human lymphoblastoma IM-9 line (ATCC; CCL-159) expressing HLA class I, as described below.

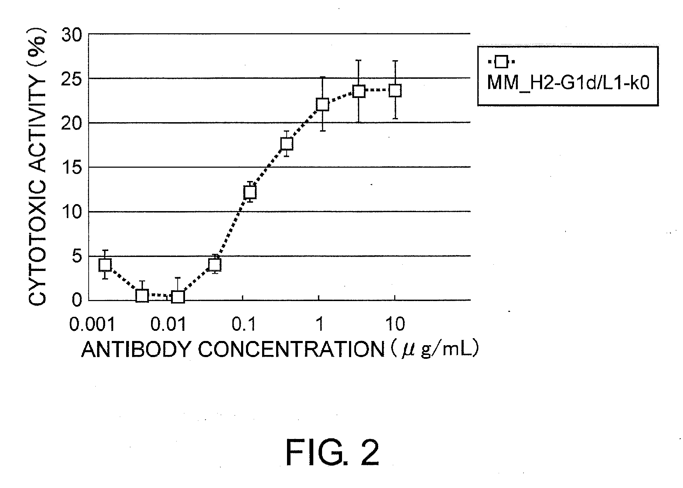

The concentration of purified H2-G1d_L1-k0 was adjusted to 20 µg/ml with medium, and a dilution series consisting of a total of nine dilutions at a dilution ratio of 3 (final concentration of 10 to 0.0015 µg/ml) was prepared using 96-well U bottom plates (BM Equipment). IM-9 cells were prepared at 1.2 x 10⁵ cells/ml in RPMI 1640 medium (SIGMA) containing 10% FBS (BOVOGEN), 2 mM L-glutamine, 1.5 g/l sodium bicarbonate, 4.5 g/l glucose, 10 mM HEPES, and 1 mM sodium pyruvate. 50 µl of the cell suspension was added to 50 µl of a diluted H2-G1d_L1-k0 solution, and the cells were cultured under 5% CO₂ at 37°C for three days in a CO₂ incubator. After culturing, 10 µl of Cell Counting Kit-8 (Dojindo) was added to each well, and the cells were incubated under 5% CO₂ at 37°C for four hours in a CO₂ incubator. Then, the absorbance was measured at 450 nm (Molecular Devices, Emax). The cell death-inducing activity of H2-G1d-L1-k0 was determined as inhibition rate (%), defining the value for a well containing medium alone (without cells and antibody) as 0%, and the value for a well containing cells alone (without antibody) as 100%. The measurement was carried out at n=5, and the average was used. As shown in Fig. 2, the result demonstrated that H2-G1d_L1-k0 suppressed the growth of the IM9 cell line in a concentration-dependent manner.

### [Example 6] Production of a deglycosylated form of the human chimeric antibody F17B1

Published documents have reported that, when human IgG1 is converted into a deglycosylated form by substituting alanine for asparagine at position 297 in the Fc domain of human IgG1, the binding to the Fc receptor on effector cells is impaired, and thus ADCC by effector cells is eliminated. Therefore, the present inventors produced a deglycosylated human chimeric antibody from the F17B1 antibody.

A heavy chain constant region (CH_N297A) (SEQ ID NO: 26) was constructed by substituting alanine for asparagine at position 297 in the Fc domain of human IgG1 according to the information in a document (Cancer Res 2008, 68, 9832-9838). The nucleotide sequence encoding the H chain, in which CH_N297A was linked to the H chain variable region (SEQ ID NO: 2) of the mouse F17B1 antibody, was inserted into a mammalian cell expression vector.

The nucleotide sequence encoding the L chain, in which the L chain constant region k0 (SEQ ID NO: 7) was linked to the L chain variable region (SEQ ID NO: 3) of the mouse F17B1 antibody, was inserted into a mammalian cell expression vector.

The antibody was expressed and purified by the method described below in Reference Example 1.

### [Example 7] Effect of the F17B1 1 antibody on prolongation of the survival period

### (1) Preparation of a mouse model for human myeloma

A mouse model for human myeloma was prepared as follows. ARH77 cells (ATCC) were prepared at 3 x 10⁷ cells/ml in RPMI 1640 medium (GIBCO BRL) supplemented with 10% fetal calf serum (GIBCO BRL). 200 µl of the above suspension of ARH77 cells (6 x 10⁶ cells/mouse) was injected into the caudal vein of SCID mice (female, six weeks old; CLEA Japan Inc.) to which 0.2 mg of an anti-asialo-GM1 antibody (Wako Pure Chemical Industries) was intraperitoneally administered in advance on the previous day.

### (2) Preparation of the antibody to be administered

On the day of administration, the concentration of the F17B1 antibody produced in Example 6 was adjusted to 1 mg/ml using PBS(-) sterilized by filtration. This sample was used for administration.

### (3) Antibody administration

On day 1 and day 8 after transplantation of ARH77 cells, the sample prepared as described in (2) was administered at 10 ml/kg once daily into the caudal vein of the mice of human myeloma model prepared as described in (1). As a negative (vehicle) control, PBS(-) sterilized by filtration was injected into the caudal vein at 10 ml/kg once daily on day 1 and day 8 in the same way. Each group included seven mice.

### (4) Results

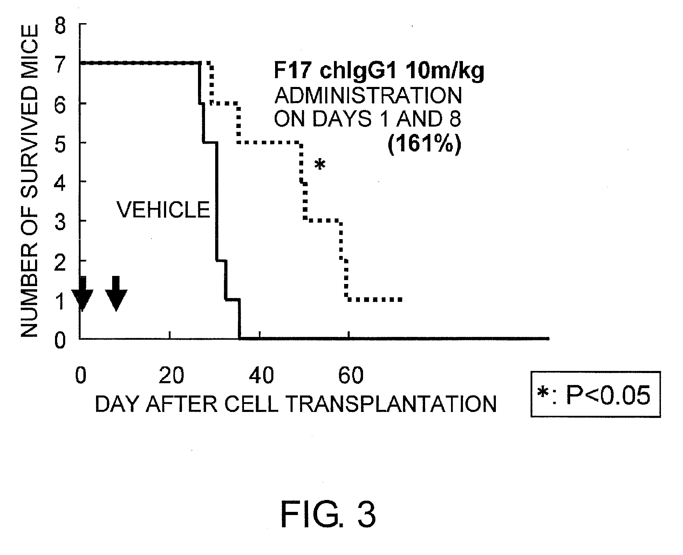

The administration of the F17B1 antibody significantly prolonged the survival period of the mouse model for human myeloma (Fig. 3).

### [Example 8] Analysis of subclasses of HLA class I to which the F17B1 antibody, 2D7 diabody, and C3B3 diabody bind

The subclasses of HLA class I to which the F17B1 antibody (F17B1 IgG) binds were analyzed using FIowPRA^{®} Specific Antibody Detection Test (One Lambda, Inc.), and were compared to the subclasses to which the 2D7 and C3B3 diabodies bind.

### (1) Methods

The F17B1 IgG (0.01 µg/ml), 2D7 diabody (1 µg/ml), and C3B3 diabody (1 µg/ml) were each added to 32 types of beads coated with 32 types of purified HLA class I antigens. After incubation at room temperature for 30 minutes, the beads were washed twice with the wash buffer attached to the kit. An FITC-conjugated F(ab7)₂ anti-human IgG was added to the F17B1 IgG-bound beads, while an FITC-conjugated anti-FLAG antibody was added to the 2D7 or C3B3 diabody-bound beads. The beads were incubated in the dark at room temperature for 30 minutes. After incubation, the beads were washed twice with the wash buffer, and fixed using PBS containing 0.5% formaldehyde. Then, the subclasses of HLA class I bound by each antibody were identified using a flow cytometer.

### (2) Results

The subclasses of HLA class I to which the F17B 1 IgG, 2D7 diabody, and C3B3 diabody bind are shown in Table 2. The F17B1 IgG was demonstrated to bind to all HLA-A except for A1, and not to any HLA-B or HLA-C. The 2D7 diabody was shown to bind to some of the HLA-A subclasses (A29, A68, and A31) and some of the HLA-B and -C subclasses. The C3B3 diabody was revealed to bind to many of the HLA-A, -B, and -C subclasses. Thus, the F17B1 IgG was demonstrated to recognize an epitope that is different from those recognized by the 2D7 and C3B3 diabodies.

**[Table 2]**

| Analysis of subclasses of HLA class I to which the F17B1 IgG, 2D7 diabody, and C3B3 diabody bind | | | |
|---|---|---|---|
| HLA class I subclass | F17B1 IgG | 2D7 diabody | C3B3 diabody |
| A1 | - | - | + |
| A2 | + | - | + |
| A3 | + | - | + |
| A11 | + | - | + |
| A23 | + | - | + |
| A24 | + | - | + |
| A25 | + | - | + |
| A26 | + | - | + |
| A29 | + | + | + |
| A30 | + | - | + |
| A31 | + | + | + |
| A32 | + | - | + |
| A33 | + | - | + |
| A34 | + | - | + |
| A68 | + | + | + |
| B7, Bw6 | - | + | + |
| B8, Bw6 | - | - | + |
| B13, Bw4 | - | - | - |
| B18, Bw6 | - | - | + |
| 827, Bw4 | - | - | + |
| B35, Bw6 | - | + | + |
| B38, Bw4 | - | - | + |
| B44, Bw4 | - | - | + |
| B45, Bw6 | - | - | + |
| B49, Bw4 | - | - | + |
| B51, Bw4 | - | + | + |
| B52, Bw4 | - | - | + |
| B55, Bw6 | - | + | + |
| B57, Bw4 | - | - | + |
| B60, Bw6 | - | - | + |
| B62, Bw6 | - | - | + |
| B65, Bw6 | - | - | + |
| Cw1 | - | + | + |
| Cw2 | - | - | + |
| Cw4 | - | - | - |
| Cw5 | - | + | + |
| Cw6 | - | + | + |
| Cw7 | - | + | + |
| Cw9 | - | + | + |
| Cw10 | - | + | + |

### [Reference Example 1] Construction of antibody expression vectors, and expression and purification of antibodies

Genes having the nucleotide sequences encoding the H and L chains of an antibody of interest were constructed by a method known to those skilled in the art using assemble PCR or the like. Amino acid substitutions were introduced by a method known to those skilled in the art using a QuikChange Site-Directed Mutagenesis Kit (Stratagene), PCR, or such. The resulting plasmid fragments were inserted into mammalian cell expression vectors to construct H and L chain expression vectors of interest. The nucleotide sequences of the constructed expression vectors were determined by a method known to those skilled in the art. Antibodies were expressed by the method described below. Cells of the human embryonic kidney cancer-derived HEK293H line (Invitrogen) were suspended in DMEM medium (Invitrogen) supplemented with 10 % Fetal Bovine Serum (Invitrogen). The cells were plated at a cell density of 5 x 10⁵ to 6 x 10⁵ cells/ml (10 ml/dish) into dishes for adherent cells (10-cm diameter; SCORNING). After the cells were cultured in a CO₂ incubator (37°C, 5% CO₂) for one whole day and night, the medium was removed by aspiration. 6.9 ml of CHO-S-SFIVI-II medium (Invitrogen) was added to the dishes. The prepared plasmids were introduced into the cells by the lipofection method. The resulting culture supernatants were collected, and then cells were removed by centrifugation (at about 2000 g and room temperature for five minutes). The culture supernatants were sterilized by filtration through a 0.22-µm filter, MILLEX(R)-GV (Millipore). Antibodies were purified from the resulting culture supernatants using rProtein A SepharoseTM Fast Flow (Amersham Biosciences) by a method known to those skilled in the art. To determine the concentration of the purified antibody, the absorbance was measured at 280 nm using a spectrophotometer. The antibody concentration was calculated from the obtained value using the absorbance coefficient calculated by the PACE method (Protein Science 1995; 4:2411-2423).

### Industrial Applicability

The present invention provides novel anti-HLA class I antibodies that have cell death-inducing activity. The antibodies are superior in exhibiting strong cell-killing activity against blood cancer cells. Thus, the antibodies are expected to have superior pharmaceutical effects as therapeutic agents against blood cancers, myelogenic immune diseases, autoimmune diseases, and such.

## Claims

1. An antibody that recognizes the same epitope recognized by an antibody that comprises a heavy chain variable region having the amino acid sequence of SEQ ID NO: 2 and a light chain variable region having the amino acid sequence of SEQ ID NO: 3.

2. The antibody of claim 1, which has cell death-inducing activity on a cell expressing HLA class I.

3. The antibody of claim 1, which has cell growth-suppressing activity on a cell expressing HLA class I.

4. The antibody of claim 1, which has cytotoxic activity on a cell expressing HLA class I.

5. The antibody of any one of claims 1 to 4, which is a chimeric antibody, humanized antibody, or human antibody.

6. A pharmaceutical composition comprising the antibody of any one of claims 1 to 5 as an active ingredient.

7. The pharmaceutical composition of claim 6, which is a cell death-inducing agent.

8. The pharmaceutical composition of claim 7, which induces cell death in a B or T cell.

9. The pharmaceutical composition of claim 6, which is a cytotoxic agent.

10. The pharmaceutical composition of claim 6, which is a cell growth-suppressing agent.

11. The pharmaceutical composition of claim 6, which is an anti-cancer agent.

12. The pharmaceutical composition of claim 11, wherein the cancer is blood cancer.

13. A method of suppressing cell growth, which comprises the step of administering the antibody of any one of claims 1 to 5 to a subject.

14. A method of treating cancer, which comprises the step of administering the antibody of any one of claims 1 to 5 to a subject.

15. The antibody of any one of claims 1 to 5 for use in suppressing cell growth or treating cancer.

16. Use of the antibody of any one of claims 1 to 5 in the manufacture of a cell growth-suppressing agent or an anti-cancer agent.


SEQUENCE LISTING

<110>  CHUGAI SEIYAKU KABUSHIKI KAISHA

<120>  Antibody to HLA classI

<130>  C1-A0912P

<160>  26

<170>  PatentIn version 3.4

<150>  JP 2009-218677
<151>  2009-09-24

<210>  1
<211>  314
<212>  PRT
<213>  Artificial

<220>
<223>  An artificially synthesized peptide sequence


<220>
<221>  VARIANT
<222>  (307)..(314)
<223>  flag tag

<400>  1

Met Ala Val Met Ala Pro Arg Thr Leu Val Leu Leu Leu Ser Gly Ala
1               5                   10                  15

Leu Ala Leu Thr Gln Thr Trp Ala Gly Ser His Ser Met Arg Tyr Phe
            20                  25                  30

Tyr Thr Ser Met Ser Arg Pro Gly Arg Gly Glu Pro Arg Phe Ile Ala
        35                  40                  45

Val Gly Tyr Val Asp Asp Thr Gln Phe Val Arg Phe Asp Ser Asp Ala
    50                  55                  60

Ala Ser Gln Arg Met Glu Pro Arg Ala Pro Trp Ile Glu Gln Glu Gly
65                  70                  75                  80

Pro Glu Tyr Trp Asp Arg Asn Thr Arg Asn Val Lys Ala Gln Ser Gln
                85                  90                  95

Thr Asp Arg Val Asp Leu Gly Thr Leu Arg Gly Tyr Tyr Asn Gln Ser
            100                 105                 110

Glu Ala Gly Ser His Thr Ile Gln Arg Met Tyr Gly Cys Asp Val Gly
        115                 120                 125

Pro Asp Gly Arg Phe Leu Arg Gly Tyr His Gln Tyr Ala Tyr Asp Gly
    130                 135                 140

Lys Asp Tyr Ile Ala Leu Lys Glu Asp Leu Arg Ser Trp Thr Ala Ala
145                 150                 155                 160

Asp Met Ala Ala Gln Thr Thr Lys His Lys Trp Glu Ala Ala His Val
                165                 170                 175

Ala Glu Gln Trp Arg Ala Tyr Leu Glu Gly Thr Cys Val Glu Trp Leu
            180                 185                 190

Arg Arg Tyr Leu Glu Asn Gly Lys Glu Thr Leu Gln Arg Thr Asp Ala
        195                 200                 205

Pro Lys Thr His Met Thr His His Ala Val Ser Asp His Glu Ala Thr
    210                 215                 220

Leu Arg Cys Trp Ala Leu Ser Phe Tyr Pro Ala Glu Ile Thr Leu Thr
225                 230                 235                 240

Trp Gln Arg Asp Gly Glu Asp Gln Thr Gln Asp Thr Glu Leu Val Glu
                245                 250                 255

Thr Arg Pro Ala Gly Asp Gly Thr Phe Gln Lys Trp Val Ala Val Val
            260                 265                 270

Val Pro Ser Gly Gln Glu Gln Arg Tyr Thr Cys His Val Gln His Glu
        275                 280                 285

Gly Leu Pro Lys Pro Leu Thr Leu Arg Trp Glu Pro Ser Ser Gln Pro
    290                 295                 300

Thr Ile Asp Tyr Lys Asp Asp Asp Asp Lys
305                 310


<210>  2
<211>  129
<212>  PRT
<213>  Mus musculus

<400>  2

Gln Val Thr Leu Lys Glu Ser Gly Pro Gly Ile Leu Gln Pro Ser Gln
1               5                   10                  15

Thr Leu Ser Leu Thr Cys Ser Phe Ser Gly Phe Ser Leu Asn Thr Phe
            20                  25                  30

Asn Met Gly Ile Gly Trp Ile Arg Gln Pro Ser Gly Lys Gly Leu Glu
        35                  40                  45

Trp Leu Ala His Ile Trp Trp Asn Asp Asp Tyr Tyr Tyr His Pro Ser
    50                  55                  60

Leu Lys Ser Arg Leu Thr Ile Ser Lys Asp Ser Ser Asn Asn Gln Val
65                  70                  75                  80

Phe Leu Lys Ile Thr Ser Val Asp Thr Ala Asp Thr Ala Thr Tyr Tyr
                85                  90                  95

Cys Ala Arg Ile Val Gly Glu Arg Ala Tyr Tyr Ser Asn Tyr Gly Phe
            100                 105                 110

Ala Trp Phe Thr Tyr Trp Gly Gln Gly Thr Leu Val Thr Val Ser Ala
        115                 120                 125

Ala

<210>  3
<211>  111
<212>  PRT
<213>  Mus musculus

<400>  3

Asp Ile Val Leu Thr Gln Ser Pro Ala Ser Leu Ala Val Ser Leu Gly
1               5                   10                  15

Gln Arg Ala Thr Ile Ser Cys Arg Ala Ser Glu Ser Val Ser Ile Arg
            20                  25                  30

Gly Thr Ser Leu Met His Trp Tyr Gln Gln Lys Pro Gly Tyr Ala Pro
        35                  40                  45

Lys Leu Leu Ile Tyr Arg Ala Ser Asn Leu Glu Ser Gly Val Pro Ala
    50                  55                  60

Arg Phe Ser Gly Ser Gly Ser Arg Thr Asp Phe Thr Leu Thr Ile Asp
65                  70                  75                  80

Pro Val Glu Ala Asp Asp Ala Ala Thr Tyr Tyr Cys Gln Gln Ser Arg
                85                  90                  95

Glu Tyr Pro Tyr Thr Phe Gly Ser Gly Thr Lys Leu Glu Ile Lys
            100                 105                 110

<210>  4
<211>  128
<212>  PRT
<213>  Artificial

<220>

<223>  An artificially synthesized peptide sequence

<400>  4

Gln Val Thr Leu Lys Glu Ser Gly Pro Thr Leu Val Lys Pro Thr Gln
1               5                   10                  15

Thr Leu Thr Leu Thr Cys Ala Phe Ser Gly Phe Ser Leu Asn Thr Phe
            20                  25                  30

Asn Met Gly Ile Gly Trp Ile Arg Gln Pro Pro Gly Lys Ala Leu Glu
        35                  40                  45

Trp Leu Ala His Ile Trp Trp Asn Asp Asp Tyr Tyr Tyr His Pro Ser
    50                  55                  60

Leu Lys Ser Arg Leu Thr Ile Ser Lys Asp Ser Ser Lys Asn Gln Val
65                  70                  75                  80

Asp Leu Thr Met Thr Asn Met Asp Pro Val Asp Thr Ala Thr Tyr Tyr
                85                  90                  95

Cys Ala Arg Ile Val Gly Glu Arg Ala Tyr Tyr Ser Asn Tyr Gly Phe
            100                 105                 110

Ala Trp Phe Thr Tyr Trp Gly Gln Gly Thr Leu Val Thr Val Ser Ser
        115                 120                 125

<210>  5
<211>  111
<212>  PRT
<213>  Artificial

<220>
<223>  An artificially synthesized peptide sequence

<400>  5

Glu Ile Val Leu Thr Gln Ser Pro Ala Thr Leu Ser Leu Ser Pro Gly
1               5                   10                  15

Glu Arg Ala Thr Leu Ser Cys Arg Ala Ser Glu Ser Val Ser Ile Arg
            20                  25                  30

Gly Thr Ser Leu Met His Trp Tyr Gln Gln Lys Pro Gly Gln Ala Pro
        35                  40                  45

Arg Leu Leu Ile Tyr Arg Ala Ser Asn Leu Glu Ser Gly Val Pro Asp
    50                  55                  60

Arg Phe Ser Gly Ser Gly Ser Gly Thr Asp Phe Thr Leu Thr Ile Asn
65                  70                  75                  80

Ser Leu Glu Ala Glu Asp Ala Ala Thr Tyr Tyr Cys Gln Gln Ser Arg
                85                  90                  95

Glu Tyr Pro Tyr Thr Phe Gly Gln Gly Thr Lys Leu Glu Ile Lys
            100                 105                 110

<210>  6
<211>  328
<212>  PRT
<213>  Homo sapiens

<400>  6

Ala Ser Thr Lys Gly Pro Ser Val Phe Pro Leu Ala Pro Ser Ser Lys
1               5                   10                  15

Ser Thr Ser Gly Gly Thr Ala Ala Leu Gly Cys Leu Val Lys Asp Tyr
            20                  25                  30

Phe Pro Glu Pro Val Thr Val Ser Trp Asn Ser Gly Ala Leu Thr Ser
        35                  40                  45

Gly Val His Thr Phe Pro Ala Val Leu Gln Ser Ser Gly Leu Tyr Ser
    50                  55                  60

Leu Ser Ser Val Val Thr Val Pro Ser Ser Ser Leu Gly Thr Gln Thr
65                  70                  75                  80

Tyr Ile Cys Asn Val Asn His Lys Pro Ser Asn Thr Lys Val Asp Lys
                85                  90                  95

Lys Val Glu Pro Lys Ser Cys Asp Lys Thr His Thr Cys Pro Pro Cys
            100                 105                 110

Pro Ala Pro Glu Leu Leu Gly Gly Pro Ser Val Phe Leu Phe Pro Pro
        115                 120                 125

Lys Pro Lys Asp Thr Leu Met Ile Ser Arg Thr Pro Glu Val Thr Cys
    130                 135                 140

Val Val Val Asp Val Ser His Glu Asp Pro Glu Val Lys Phe Asn Trp
145                 150                 155                 160

Tyr Val Asp Gly Val Glu Val His Asn Ala Lys Thr Lys Pro Arg Glu
                165                 170                 175

Glu Gln Tyr Asn Ser Thr Tyr Arg Val Val Ser Val Leu Thr Val Leu
            180                 185                 190

His Gln Asp Trp Leu Asn Gly Lys Glu Tyr Lys Cys Lys Val Ser Asn

195                 200                 205

Lys Ala Leu Pro Ala Pro Ile Glu Lys Thr Ile Ser Lys Ala Lys Gly
    210                 215                 220

Gln Pro Arg Glu Pro Gln Val Tyr Thr Leu Pro Pro Ser Arg Asp Glu
225                 230                 235                 240

Leu Thr Lys Asn Gln Val Ser Leu Thr Cys Leu Val Lys Gly Phe Tyr
                245                 250                 255

Pro Ser Asp Ile Ala Val Glu Trp Glu Ser Asn Gly Gln Pro Glu Asn
            260                 265                 270

Asn Tyr Lys Thr Thr Pro Pro Val Leu Asp Ser Asp Gly Ser Phe Phe
        275                 280                 285

Leu Tyr Ser Lys Leu Thr Val Asp Lys Ser Arg Trp Gln Gln Gly Asn
    290                 295                 300

Val Phe Ser Cys Ser Val Met His Glu Ala Leu His Asn His Tyr Thr
305                 310                 315                 320

Gln Lys Ser Leu Ser Leu Ser Pro
                325

<210>  7
<211>  107
<212>  PRT
<213>  Homo sapiens

<400>  7

<210> 8
<211> 456
<212> PRT
<213> Artificial

<220>
<223> An artificially synthesized peptide sequence

<400> 8

Gln Val Thr Leu Lys Glu Ser Gly Pro Thr Leu Val Lys Pro Thr Gln
1               5                   10                  15

Thr Leu Thr Leu Thr Cys Ala Phe Ser Gly Phe Ser Leu Asn Thr Phe
            20                  25                  30

Asn Met Gly Ile Gly Trp Ile Arg Gln Pro Pro Gly Lys Ala Leu Glu
        35                  40                  45

Trp Leu Ala His Ile Trp Trp Asn Asp Asp Tyr Tyr Tyr His Pro Ser
    50                  55                  60

Leu Lys Ser Arg Leu Thr Ile Ser Lys Asp Ser Ser Lys Asn Gln Val
65                  70                  75                  80

Asp Leu Thr Met Thr Asn Met Asp Pro Val Asp Thr Ala Thr Tyr Tyr
                85                  90                  95

Cys Ala Arg Ile Val Gly Glu Arg Ala Tyr Tyr Ser Asn Tyr Gly Phe
            100                 105                 110

Ala Trp Phe Thr Tyr Trp Gly Gln Gly Thr Leu Val Thr Val Ser Ser
        115                 120                 125

Ala Ser Thr Lys Gly Pro Ser Val Phe Pro Leu Ala Pro Ser Ser Lys
    130                 135                 140

Ser Thr Ser Gly Gly Thr Ala Ala Leu Gly Cys Leu Val Lys Asp Tyr
145                 150                 155                 160

Phe Pro Glu Pro Val Thr Val Ser Trp Asn Ser Gly Ala Leu Thr Ser
                165                 170                 175

Gly Val His Thr Phe Pro Ala Val Leu Gln Ser Ser Gly Leu Tyr Ser
            180                 185                 190

Leu Ser Ser Val Val Thr Val Pro Ser Ser Ser Leu Gly Thr Gln Thr
        195                 200                 205

Tyr Ile Cys Asn Val Asn His Lys Pro Ser Asn Thr Lys Val Asp Lys
    210                 215                 220

Lys Val Glu Pro Lys Ser Cys Asp Lys Thr His Thr Cys Pro Pro Cys
225                 230                 235                 240

Pro Ala Pro Glu Leu Leu Gly Gly Pro Ser Val Phe Leu Phe Pro Pro
                245                 250                 255

Lys Pro Lys Asp Thr Leu Met Ile Ser Arg Thr Pro Glu Val Thr Cys
            260                 265                 270

Val Val Val Asp Val Ser His Glu Asp Pro Glu Val Lys Phe Asn Trp
        275                 280                 285

Tyr Val Asp Gly Val Glu Val His Asn Ala Lys Thr Lys Pro Arg Glu
    290                 295                 300

Glu Gln Tyr Asn Ser Thr Tyr Arg Val Val Ser Val Leu Thr Val Leu
305                 310                 315                 320

His Gln Asp Trp Leu Asn Gly Lys Glu Tyr Lys Cys Lys Val Ser Asn
                325                 330                 335

Lys Ala Leu Pro Ala Pro Ile Glu Lys Thr Ile Ser Lys Ala Lys Gly
            340                 345                 350

Gln Pro Arg Glu Pro Gln Val Tyr Thr Leu Pro Pro Ser Arg Asp Glu
        355                 360                 365

Leu Thr Lys Asn Gln Val Ser Leu Thr Cys Leu Val Lys Gly Phe Tyr
    370                 375                 380

Pro Ser Asp Ile Ala Val Glu Trp Glu Ser Asn Gly Gln Pro Glu Asn
385                 390                 395                 400

Asn Tyr Lys Thr Thr Pro Pro Val Leu Asp Ser Asp Gly Ser Phe Phe
                405                 410                 415

Leu Tyr Ser Lys Leu Thr Val Asp Lys Ser Arg Trp Gln Gln Gly Asn
            420                 425                 430

Val Phe Ser Cys Ser Val Met His Glu Ala Leu His Asn His Tyr Thr
        435                 440                 445

Gln Lys Ser Leu Ser Leu Ser Pro
    450                 455

<210>  9
<211>  218
<212>  PRT
<213>  Artificial

<220>
<223>  An artificially synthesized peptide sequence

<400>  9

Glu Ile Val Leu Thr Gln Ser Pro Ala Thr Leu Ser Leu Ser Pro Gly
1               5                   10                  15

Glu Arg Ala Thr Leu Ser Cys Arg Ala Ser Glu Ser Val Ser Ile Arg
            20                  25                  30

Gly Thr Ser Leu Met His Trp Tyr Gln Gln Lys Pro Gly Gln Ala Pro
        35                  40                  45

Arg Leu Leu Ile Tyr Arg Ala Ser Asn Leu Glu Ser Gly Val Pro Asp
    50                  55                  60

Arg Phe Ser Gly Ser Gly Ser Gly Thr Asp Phe Thr Leu Thr Ile Asn
65                  70                  75                  80

Ser Leu Glu Ala Glu Asp Ala Ala Thr Tyr Tyr Cys Gln Gln Ser Arg
                85                  90                  95

Glu Tyr Pro Tyr Thr Phe Gly Gln Gly Thr Lys Leu Glu Ile Lys Arg
            100                 105                 110

Thr Val Ala Ala Pro Ser Val Phe Ile Phe Pro Pro Ser Asp Glu Gln
        115                 120                 125

Leu Lys Ser Gly Thr Ala Ser Val Val Cys Leu Leu Asn Asn Phe Tyr
    130                 135                 140

Pro Arg Glu Ala Lys Val Gln Trp Lys Val Asp Asn Ala Leu Gln Ser
145                 150                 155                 160

Gly Asn Ser Gln Glu Ser Val Thr Glu Gln Asp Ser Lys Asp Ser Thr
                165                 170                 175

Tyr Ser Leu Ser Ser Thr Leu Thr Leu Ser Lys Ala Asp Tyr Glu Lys
            180                 185                 190

His Lys Val Tyr Ala Cys Glu Val Thr His Gln Gly Leu Ser Ser Pro
        195                 200                 205

Val Thr Lys Ser Phe Asn Arg Gly Glu Cys
    210                 215

<210>  10
<211>  30
<212>  PRT
<213>  Homo sapiens

<400>  10

Gln Val Thr Leu Lys Glu Ser Gly Pro Thr Leu Val Lys Pro Thr Gln
1               5                   10                  15

Thr Leu Thr Leu Thr Cys Ala Phe Ser Gly Phe Ser Leu Asn
            20                  25                  30

<210>  11
<211>  14
<212>  PRT
<213>  Homo sapiens

<400>  11

Trp Ile Arg Gln Pro Pro Gly Lys Ala Leu Glu Trp Leu Ala
1               5                   10

<210>  12
<211>  32
<212>  PRT
<213>  Homo sapiens

<400>  12

Arg Leu Thr Ile Ser Lys Asp Ser Ser Lys Asn Gln Val Asp Leu Thr
1               5                   10                  15

Met Thr Asn Met Asp Pro Val Asp Thr Ala Thr Tyr Tyr Cys Ala Arg
            20                  25                  30

<210>  13
<211>  11
<212>  PRT
<213>  Homo sapiens

<400>  13

Trp Gly Gln Gly Thr Leu Val Thr Val Ser Ser
1               5                   10

<210>  14
<211>  23
<212>  PRT
<213>  Homo sapiens

<400>  14

Glu Ile Val Leu Thr Gln Ser Pro Ala Thr Leu Ser Leu Ser Pro Gly
1               5                   10                  15

Glu Arg Ala Thr Leu Ser Cys
            20

<210>  15
<211>  15
<212>  PRT
<213>  Homo sapiens

<400>  15

Trp Tyr Gln Gln Lys Pro Gly Gln Ala Pro Arg Leu Leu Ile Tyr
1               5                   10                  15

<210>  16
<211>  32
<212>  PRT
<213>  Homo sapiens

<400>  16

Gly Val Pro Asp Arg Phe Ser Gly Ser Gly Ser Gly Thr Asp Phe Thr
1               5                   10                  15

Leu Thr Ile Asn Ser Leu Glu Ala Glu Asp Ala Ala Thr Tyr Tyr Cys
            20                  25                  30

<210>  17
<211>  10
<212>  PRT
<213>  Homo sapiens

<400>  17

Phe Gly Gln Gly Thr Lys Leu Glu Ile Lys
1               5                   10

<210>  18
<211>  32
<212>  PRT
<213>  Homo sapiens

<400>  18

Gly Val Pro Asp Arg Phe Ser Gly Ser Gly Ser Gly Thr Asp Phe Thr
1               5                   10                  15

Leu Lys Ile Ser Arg Val Glu Ala Glu Asp Val Gly Val Tyr Tyr Cys
            20                  25                  30

<210>  19
<211>  32
<212>  PRT
<213>  Homo sapiens

<400>  19

Gly Val Pro Ser Arg Phe Ser Gly Ser Gly Ser Gly Thr Asp Phe Thr
1               5                   10                  15

Leu Thr Ile Asn Ser Leu Glu Ala Glu Asp Ala Ala Thr Tyr Tyr Cys
            20                  25                  30

<210>  20
<211>  7
<212>  PRT
<213>  Artificial

<220>
<223>  An artificially synthesized peptide sequence

<400>  20

Thr Phe Asn Met Gly Ile Gly
1               5

<210>  21
<211>  16
<212>  PRT
<213>  Artificial

<220>
<223>  An artificially synthesized peptide sequence

<400>  21

His Ile Trp Trp Asn Asp Asp Tyr Tyr Tyr His Pro Ser Leu Lys Ser
1               5                   10                  15

<210>  22
<211>  18
<212>  PRT
<213>  Artificial

<220>
<223>  An artificially synthesized peptide sequence

<400>  22

Ile Val Gly Glu Arg Ala Tyr Tyr Ser Asn Tyr Gly Phe Ala Trp Phe
1               5                   10                  15

Thr Tyr

<210>  23
<211>  15
<212>  PRT
<213>  Artificial

<220>
<223>  An artificially synthesized peptide sequence

<400>  23

Arg Ala Ser Glu Ser Val Ser Ile Arg Gly Thr Ser Leu Met His
1               5                   10                  15

<210>  24
<211>  7
<212>  PRT
<213>  Artificial

<220>
<223>  An artificially synthesized peptide sequence

<400>  24

Arg Ala Ser Asn Leu Glu Ser
1               5


<210>  25
<211>  9
<212>  PRT
<213>  Artificial

<220>
<223>  An artificially synthesized peptide sequence

<400>  25

Gln Gln Ser Arg Glu Tyr Pro Tyr Thr
1               5


<210>  26
<211>  993
<212>  DNA
<213>  Artificial

<220>
<223>  An artificially synthesized origonucleotide sequence

<400>  26
gctagcacca agggcccatc ggtcttcccc ctggcaccct cctccaagag cacctctggg      60

ggcacagcgg ccctgggctg cctggtcaag gactacttcc ccgaaccggt gacggtgtcg     120

tggaactcag gcgccctgac cagcggcgtg cacaccttcc cggctgtcct acagtcctca     180

ggactctact ccctcagcag cgtggtgacc gtgccctcca gcagcttggg cacccagacc     240

tacatctgca acgtgaatca caagcccagc aacaccaagg tggacaagaa agttgagccc     300

aaatcttgtg acaaaactca cacatgccca ccgtgcccag cacctgaact cctgggggga     360

ccgtcagtct tcctcttccc cccaaaaccc aaggacaccc tcatgatctc ccggacccct     420

gaggtcacat gcgtggtggt ggacgtgagc cacgaagacc ctgaggtcaa gttcaactgg     480

tacgtggacg gcgtggaggt gcataatgcc aagacaaagc cgcgggagga gcagtacgcc     540

agcacgtacc gtgtggtcag cgtcctcacc gtcctgcacc aggactggct gaatggcaag     600

gagtacaagt gcaaggtctc caacaaagcc ctcccagccc ccatcgagaa aaccatctcc     660

aaagccaaag ggcagccccg agaaccacag gtgtacaccc tgcccccatc ccgggatgag     720

ctgaccaaga accaggtcag cctgacctgc ctggtcaaag gcttctatcc cagcgacatc     780

gccgtggagt gggagagcaa tgggcagccg gagaacaact acaagaccac gcctcccgtg     840

ctggactccg acggctcctt cttcctctac agcaagctca ccgtggacaa gagcaggtgg 900

cagcagggga acgtcttctc atgctccgtg atgcatgagg ctctgcacaa ccactacacg 960

cagaagagcc tctccctgtc tccgggtaaa tga 993

SEQUENCE LISTING

<110>  CHUGAI SEIYAKU KABUSHIKI KAISHA

<120>  ANTIBODY CAPABLE OF RECOGNIZING HLA CLASS I

<130>  U1488 EP S3

<140>  EP 10 81 8832.7
<141>  2010-09-24

<150>  JP 2009-218677
<151>  2009-09-24

<160>  26

<170>  PatentIn version 3.4

<210>  1
<211>  314
<212>  PRT
<213>  Artificial

<220>
<223>  An artificially synthesized peptide sequence


<220>
<221>  VARIANT
<222>  (307)..(314)
<223>  flag tag

<400>  1

Met Ala Val Met Ala Pro Arg Thr Leu Val Leu Leu Leu Ser Gly Ala
1               5                   10                  15


Leu Ala Leu Thr Gln Thr Trp Ala Gly Ser His Ser Met Arg Tyr Phe
            20                  25                  30


Tyr Thr Ser Met Ser Arg Pro Gly Arg Gly Glu Pro Arg Phe Ile Ala
        35                  40                  45


Val Gly Tyr Val Asp Asp Thr Gln Phe Val Arg Phe Asp Ser Asp Ala
    50                  55                  60


Ala Ser Gln Arg Met Glu Pro Arg Ala Pro Trp Ile Glu Gln Glu Gly
65                  70                  75                  80


Pro Glu Tyr Trp Asp Arg Asn Thr Arg Asn Val Lys Ala Gln Ser Gln
                85                  90                  95


Thr Asp Arg Val Asp Leu Gly Thr Leu Arg Gly Tyr Tyr Asn Gln Ser
            100                 105                 110


Glu Ala Gly Ser His Thr Ile Gln Arg Met Tyr Gly Cys Asp Val Gly
        115                 120                 125

Pro Asp Gly Arg Phe Leu Arg Gly Tyr His Gln Tyr Ala Tyr Asp Gly
    130                 135                 140

Lys Asp Tyr Ile Ala Leu Lys Glu Asp Leu Arg Ser Trp Thr Ala Ala
145                 150                 155                 160

Asp Met Ala Ala Gln Thr Thr Lys His Lys Trp Glu Ala Ala His Val
                165                 170                 175

Ala Glu Gln Trp Arg Ala Tyr Leu Glu Gly Thr Cys Val Glu Trp Leu
            180                 185                 190

Arg Arg Tyr Leu Glu Asn Gly Lys Glu Thr Leu Gln Arg Thr Asp Ala
        195                 200                 205

Pro Lys Thr His Met Thr His His Ala Val Ser Asp His Glu Ala Thr
    210                 215                 220

Leu Arg Cys Trp Ala Leu Ser Phe Tyr Pro Ala Glu Ile Thr Leu Thr
225                 230                 235                 240

Trp Gln Arg Asp Gly Glu Asp Gln Thr Gln Asp Thr Glu Leu Val Glu
                245                 250                 255

Thr Arg Pro Ala Gly Asp Gly Thr Phe Gln Lys Trp Val Ala Val Val
            260                 265                 270

Val Pro Ser Gly Gln Glu Gln Arg Tyr Thr Cys His Val Gln His Glu
        275                 280                 285

Gly Leu Pro Lys Pro Leu Thr Leu Arg Trp Glu Pro Ser Ser Gln Pro
    290                 295                 300

Thr Ile Asp Tyr Lys Asp Asp Asp Asp Lys
305                 310

<210>  2
<211>  129
<212>  PRT
<213>  Mus musculus

<400>  2

Gln Val Thr Leu Lys Glu Ser Gly Pro Gly Ile Leu Gln Pro Ser Gln
1               5                   10                  15

Thr Leu Ser Leu Thr Cys Ser Phe Ser Gly Phe Ser Leu Asn Thr Phe
            20                  25                  30

Asn Met Gly Ile Gly Trp Ile Arg Gln Pro Ser Gly Lys Gly Leu Glu

35                  40                  45

Trp Leu Ala His Ile Trp Trp Asn Asp Asp Tyr Tyr Tyr His Pro Ser
    50                  55                  60

Leu Lys Ser Arg Leu Thr Ile Ser Lys Asp Ser Ser Asn Asn Gln Val
65                  70                  75                  80

Phe Leu Lys Ile Thr Ser Val Asp Thr Ala Asp Thr Ala Thr Tyr Tyr
                85                  90                  95

Cys Ala Arg Ile Val Gly Glu Arg Ala Tyr Tyr Ser Asn Tyr Gly Phe
            100                 105                 110

Ala Trp Phe Thr Tyr Trp Gly Gln Gly Thr Leu Val Thr Val Ser Ala
        115                 120                 125

Ala

<210>  3
<211>  111
<212>  PRT
<213>  Mus musculus

<400>  3

Asp Ile Val Leu Thr Gln Ser Pro Ala Ser Leu Ala Val Ser Leu Gly
1               5                   10                  15

Gln Arg Ala Thr Ile Ser Cys Arg Ala Ser Glu Ser Val Ser Ile Arg
            20                  25                  30

Gly Thr Ser Leu Met His Trp Tyr Gln Gln Lys Pro Gly Tyr Ala Pro
        35                  40                  45

Lys Leu Leu Ile Tyr Arg Ala Ser Asn Leu Glu Ser Gly Val Pro Ala
    50                  55                  60

Arg Phe Ser Gly Ser Gly Ser Arg Thr Asp Phe Thr Leu Thr Ile Asp
65                  70                  75                  80

Pro Val Glu Ala Asp Asp Ala Ala Thr Tyr Tyr Cys Gln Gln Ser Arg
                85                  90                  95

Glu Tyr Pro Tyr Thr Phe Gly Ser Gly Thr Lys Leu Glu Ile Lys
            100                 105                 110

<210>  4
<211>  128
<212>  PRT

<213>  Artificial

<220>
<223>  An artificially synthesized peptide sequence

<400>  4

Gln Val Thr Leu Lys Glu Ser Gly Pro Thr Leu Val Lys Pro Thr Gln
1               5                   10                  15

Thr Leu Thr Leu Thr Cys Ala Phe Ser Gly Phe Ser Leu Asn Thr Phe
            20                  25                  30

Asn Met Gly Ile Gly Trp Ile Arg Gln Pro Pro Gly Lys Ala Leu Glu
        35                  40                  45

Trp Leu Ala His Ile Trp Trp Asn Asp Asp Tyr Tyr Tyr His Pro Ser
    50                  55                  60

Leu Lys Ser Arg Leu Thr Ile Ser Lys Asp Ser Ser Lys Asn Gln Val
65                  70                  75                  80

Asp Leu Thr Met Thr Asn Met Asp Pro Val Asp Thr Ala Thr Tyr Tyr
                85                  90                  95

Cys Ala Arg Ile Val Gly Glu Arg Ala Tyr Tyr Ser Asn Tyr Gly Phe
            100                 105                 110

Ala Trp Phe Thr Tyr Trp Gly Gln Gly Thr Leu Val Thr Val Ser Ser
        115                 120                 125


<210>  5
<211>  111
<212>  PRT
<213>  Artificial

<220>
<223>  An artificially synthesized peptide sequence

<400>  5

Glu Ile Val Leu Thr Gln Ser Pro Ala Thr Leu Ser Leu Ser Pro Gly
1               5                   10                  15

Glu Arg Ala Thr Leu Ser Cys Arg Ala Ser Glu Ser Val Ser Ile Arg
            20                  25                  30

Gly Thr Ser Leu Met His Trp Tyr Gln Gln Lys Pro Gly Gln Ala Pro
        35                  40                  45

Arg Leu Leu Ile Tyr Arg Ala Ser Asn Leu Glu Ser Gly Val Pro Asp
    50                  55                  60

Arg Phe Ser Gly Ser Gly Ser Gly Thr Asp Phe Thr Leu Thr Ile Asn
65                  70                  75                  80

Ser Leu Glu Ala Glu Asp Ala Ala Thr Tyr Tyr Cys Gln Gln Ser Arg
                85                  90                  95

Glu Tyr Pro Tyr Thr Phe Gly Gln Gly Thr Lys Leu Glu Ile Lys
            100                 105                 110

<210>  6
<211>  328
<212>  PRT
<213>  Homo sapiens

<400>  6

Ala Ser Thr Lys Gly Pro Ser Val Phe Pro Leu Ala Pro Ser Ser Lys
1               5                   10                  15

Ser Thr Ser Gly Gly Thr Ala Ala Leu Gly Cys Leu Val Lys Asp Tyr
            20                  25                  30

Phe Pro Glu Pro Val Thr Val Ser Trp Asn Ser Gly Ala Leu Thr Ser
        35                  40                  45

Gly Val His Thr Phe Pro Ala Val Leu Gln Ser Ser Gly Leu Tyr Ser
    50                  55                  60

Leu Ser Ser Val Val Thr Val Pro Ser Ser Ser Leu Gly Thr Gln Thr
65                  70                  75                  80

Tyr Ile Cys Asn Val Asn His Lys Pro Ser Asn Thr Lys Val Asp Lys
                85                  90                  95

Lys Val Glu Pro Lys Ser Cys Asp Lys Thr His Thr Cys Pro Pro Cys
            100                 105                 110

Pro Ala Pro Glu Leu Leu Gly Gly Pro Ser Val Phe Leu Phe Pro Pro
        115                 120                 125

Lys Pro Lys Asp Thr Leu Met Ile Ser Arg Thr Pro Glu Val Thr Cys
    130                 135                 140

Val Val Val Asp Val Ser His Glu Asp Pro Glu Val Lys Phe Asn Trp
145                 150                 155                 160

Tyr Val Asp Gly Val Glu Val His Asn Ala Lys Thr Lys Pro Arg Glu
                165                 170                 175

Glu Gln Tyr Asn Ser Thr Tyr Arg Val Val Ser Val Leu Thr Val Leu
            180                 185                 190

His Gln Asp Trp Leu Asn Gly Lys Glu Tyr Lys Cys Lys Val Ser Asn
        195                 200                 205

Lys Ala Leu Pro Ala Pro Ile Glu Lys Thr Ile Ser Lys Ala Lys Gly
    210                 215                 220

Gln Pro Arg Glu Pro Gln Val Tyr Thr Leu Pro Pro Ser Arg Asp Glu
225                 230                 235                 240

Leu Thr Lys Asn Gln Val Ser Leu Thr Cys Leu Val Lys Gly Phe Tyr
                245                 250                 255

Pro Ser Asp Ile Ala Val Glu Trp Glu Ser Asn Gly Gln Pro Glu Asn
            260                 265                 270

Asn Tyr Lys Thr Thr Pro Pro Val Leu Asp Ser Asp Gly Ser Phe Phe
        275                 280                 285

Leu Tyr Ser Lys Leu Thr Val Asp Lys Ser Arg Trp Gln Gln Gly Asn
    290                 295                 300

Val Phe Ser Cys Ser Val Met His Glu Ala Leu His Asn His Tyr Thr
305                 310                 315                 320

Gln Lys Ser Leu Ser Leu Ser Pro
                325

<210> 7
<211> 107
<212> PRT
<213> Homo sapiens

<400> 7

<210>  8
<211>  456
<212>  PRT
<213>  Artificial

<220>
<223>  An artificially synthesized peptide sequence

<400>  8

Gln Val Thr Leu Lys Glu Ser Gly Pro Thr Leu Val Lys Pro Thr Gln
1               5                   10                  15

Thr Leu Thr Leu Thr Cys Ala Phe Ser Gly Phe Ser Leu Asn Thr Phe
            20                  25                  30

Asn Met Gly Ile Gly Trp Ile Arg Gln Pro Pro Gly Lys Ala Leu Glu
        35                  40                  45

Trp Leu Ala His Ile Trp Trp Asn Asp Asp Tyr Tyr Tyr His Pro Ser
    50                  55                  60

Leu Lys Ser Arg Leu Thr Ile Ser Lys Asp Ser Ser Lys Asn Gln Val
65                  70                  75                  80

Asp Leu Thr Met Thr Asn Met Asp Pro Val Asp Thr Ala Thr Tyr Tyr
                85                  90                  95

Cys Ala Arg Ile Val Gly Glu Arg Ala Tyr Tyr Ser Asn Tyr Gly Phe
            100                 105                 110

Ala Trp Phe Thr Tyr Trp Gly Gln Gly Thr Leu Val Thr Val Ser Ser
        115                 120                 125

Ala Ser Thr Lys Gly Pro Ser Val Phe Pro Leu Ala Pro Ser Ser Lys
    130                 135                 140

Ser Thr Ser Gly Gly Thr Ala Ala Leu Gly Cys Leu Val Lys Asp Tyr
145                 150                 155                 160

Phe Pro Glu Pro Val Thr Val Ser Trp Asn Ser Gly Ala Leu Thr Ser
                165                 170                 175

Gly Val His Thr Phe Pro Ala Val Leu Gln Ser Ser Gly Leu Tyr Ser
            180                 185                 190

Leu Ser Ser Val Val Thr Val Pro Ser Ser Ser Leu Gly Thr Gln Thr
        195                 200                 205

Tyr Ile Cys Asn Val Asn His Lys Pro Ser Asn Thr Lys Val Asp Lys
    210                 215                 220

Lys Val Glu Pro Lys Ser Cys Asp Lys Thr His Thr Cys Pro Pro Cys
225                 230                 235                 240

Pro Ala Pro Glu Leu Leu Gly Gly Pro Ser Val Phe Leu Phe Pro Pro
                245                 250                 255

Lys Pro Lys Asp Thr Leu Met Ile Ser Arg Thr Pro Glu Val Thr Cys
            260                 265                 270

Val Val Val Asp Val Ser His Glu Asp Pro Glu Val Lys Phe Asn Trp
        275                 280                 285

Tyr Val Asp Gly Val Glu Val His Asn Ala Lys Thr Lys Pro Arg Glu
    290                 295                 300

Glu Gln Tyr Asn Ser Thr Tyr Arg Val Val Ser Val Leu Thr Val Leu
305                 310                 315                 320

His Gln Asp Trp Leu Asn Gly Lys Glu Tyr Lys Cys Lys Val Ser Asn
                325                 330                 335

Lys Ala Leu Pro Ala Pro Ile Glu Lys Thr Ile Ser Lys Ala Lys Gly
            340                 345                 350

Gln Pro Arg Glu Pro Gln Val Tyr Thr Leu Pro Pro Ser Arg Asp Glu
        355                 360                 365

Leu Thr Lys Asn Gln Val Ser Leu Thr Cys Leu Val Lys Gly Phe Tyr
    370                 375                 380

Pro Ser Asp Ile Ala Val Glu Trp Glu Ser Asn Gly Gln Pro Glu Asn
385                 390                 395                 400

Asn Tyr Lys Thr Thr Pro Pro Val Leu Asp Ser Asp Gly Ser Phe Phe
                405                 410                 415

Leu Tyr Ser Lys Leu Thr Val Asp Lys Ser Arg Trp Gln Gln Gly Asn
            420                 425                 430

Val Phe Ser Cys Ser Val Met His Glu Ala Leu His Asn His Tyr Thr
        435                 440                 445

Gln Lys Ser Leu Ser Leu Ser Pro
    450                 455

<210>  9
<211>  218
<212>  PRT
<213>  Artificial

<220>
<223>  An artificially synthesized peptide sequence

<400>  9

Glu Ile Val Leu Thr Gln Ser Pro Ala Thr Leu Ser Leu Ser Pro Gly
1               5                   10                  15

Glu Arg Ala Thr Leu Ser Cys Arg Ala Ser Glu Ser Val Ser Ile Arg
            20                  25                  30

Gly Thr Ser Leu Met His Trp Tyr Gln Gln Lys Pro Gly Gln Ala Pro
        35                  40                  45

Arg Leu Leu Ile Tyr Arg Ala Ser Asn Leu Glu Ser Gly Val Pro Asp
    50                  55                  60

Arg Phe Ser Gly Ser Gly Ser Gly Thr Asp Phe Thr Leu Thr Ile Asn
65                  70                  75                  80

Ser Leu Glu Ala Glu Asp Ala Ala Thr Tyr Tyr Cys Gln Gln Ser Arg
                85                  90                  95

Glu Tyr Pro Tyr Thr Phe Gly Gln Gly Thr Lys Leu Glu Ile Lys Arg
            100                 105                 110

Thr Val Ala Ala Pro Ser Val Phe Ile Phe Pro Pro Ser Asp Glu Gln
        115                 120                 125

Leu Lys Ser Gly Thr Ala Ser Val Val Cys Leu Leu Asn Asn Phe Tyr
    130                 135                 140

Pro Arg Glu Ala Lys Val Gln Trp Lys Val Asp Asn Ala Leu Gln Ser
145                 150                 155                 160

Gly Asn Ser Gln Glu Ser Val Thr Glu Gln Asp Ser Lys Asp Ser Thr
                165                 170                 175

Tyr Ser Leu Ser Ser Thr Leu Thr Leu Ser Lys Ala Asp Tyr Glu Lys
            180                 185                 190

His Lys Val Tyr Ala Cys Glu Val Thr His Gln Gly Leu Ser Ser Pro
        195                 200                 205

Val Thr Lys Ser Phe Asn Arg Gly Glu Cys
    210                 215

<210>  10
<211>  30
<212>  PRT
<213>  Homo sapiens

<400>  10

Gln Val Thr Leu Lys Glu Ser Gly Pro Thr Leu Val Lys Pro Thr Gln
1               5                   10                  15

Thr Leu Thr Leu Thr Cys Ala Phe Ser Gly Phe Ser Leu Asn
            20                  25                  30

<210>  11
<211>  14
<212>  PRT
<213>  Homo sapiens

<400>  11

Trp Ile Arg Gln Pro Pro Gly Lys Ala Leu Glu Trp Leu Ala
1               5                   10

<210>  12
<211>  32
<212>  PRT
<213>  Homo sapiens

<400>  12

Arg Leu Thr Ile Ser Lys Asp Ser Ser Lys Asn Gln Val Asp Leu Thr
1               5                   10                  15

Met Thr Asn Met Asp Pro Val Asp Thr Ala Thr Tyr Tyr Cys Ala Arg
            20                  25                  30

<210>  13
<211>  11
<212>  PRT
<213>  Homo sapiens

<400>  13

Trp Gly Gln Gly Thr Leu Val Thr Val Ser Ser
1               5                   10

<210>  14
<211>  23
<212>  PRT
<213>  Homo sapiens

<400>  14

Glu Ile Val Leu Thr Gln Ser Pro Ala Thr Leu Ser Leu Ser Pro Gly

1               5                   10                  15

Glu Arg Ala Thr Leu Ser Cys
            20

<210>  15
<211>  15
<212>  PRT
<213>  Homo sapiens

<400>  15

Trp Tyr Gln Gln Lys Pro Gly Gln Ala Pro Arg Leu Leu Ile Tyr
1               5                   10                  15

<210>  16
<211>  32
<212>  PRT
<213>  Homo sapiens

<400>  16

Gly Val Pro Asp Arg Phe Ser Gly Ser Gly Ser Gly Thr Asp Phe Thr
1               5                   10                  15

Leu Thr Ile Asn Ser Leu Glu Ala Glu Asp Ala Ala Thr Tyr Tyr Cys
            20                  25                  30

<210>  17
<211>  10
<212>  PRT
<213>  Homo sapiens

<400>  17

Phe Gly Gln Gly Thr Lys Leu Glu Ile Lys
1               5                   10

<210>  18
<211>  32
<212>  PRT
<213>  Homo sapiens

<400>  18

Gly Val Pro Asp Arg Phe Ser Gly Ser Gly Ser Gly Thr Asp Phe Thr
1               5                   10                  15

Leu Lys Ile Ser Arg Val Glu Ala Glu Asp Val Gly Val Tyr Tyr Cys
            20                  25                  30

<210>  19
<211>  32
<212>  PRT
<213>  Homo sapiens

<400>  19

Gly Val Pro Ser Arg Phe Ser Gly Ser Gly Ser Gly Thr Asp Phe Thr
1               5                   10                  15

Leu Thr Ile Asn Ser Leu Glu Ala Glu Asp Ala Ala Thr Tyr Tyr Cys
            20                  25                  30

<210>  20
<211>  7
<212>  PRT
<213>  Artificial

<220>
<223>  An artificially synthesized peptide sequence

<400>  20

Thr Phe Asn Met Gly Ile Gly
1               5

<210>  21
<211>  16
<212>  PRT
<213>  Artificial

<220>
<223>  An artificially synthesized peptide sequence

<400>  21

His Ile Trp Trp Asn Asp Asp Tyr Tyr Tyr His Pro Ser Leu Lys Ser
1               5                   10                  15

<210>  22
<211>  18
<212>  PRT
<213>  Artificial

<220>
<223>  An artificially synthesized peptide sequence

<400>  22

Ile Val Gly Glu Arg Ala Tyr Tyr Ser Asn Tyr Gly Phe Ala Trp Phe
1               5                   10                  15

Thr Tyr

<210>  23
<211>  15
<212>  PRT
<213>  Artificial

<220>
<223>  An artificially synthesized peptide sequence

<400>  23

Arg Ala Ser Glu Ser Val Ser Ile Arg Gly Thr Ser Leu Met His
1               5                   10                  15

<210>  24
<211>  7
<212>  PRT
<213>  Artificial

<220>
<223>  An artificially synthesized peptide sequence

<400>  24

Arg Ala Ser Asn Leu Glu Ser
1               5

<210>  25
<211>  9
<212>  PRT
<213>  Artificial

<220>
<223>  An artificially synthesized peptide sequence

<400>  25

Gln Gln Ser Arg Glu Tyr Pro Tyr Thr
1               5

<210>  26
<211>  993
<212>  DNA
<213>  Artificial

<220>
<223>  An artificially synthesized origonucleotide sequence

<400>  26
gctagcacca agggcccatc ggtcttcccc ctggcaccct cctccaagag cacctctggg      60

ggcacagcgg ccctgggctg cctggtcaag gactacttcc ccgaaccggt gacggtgtcg     120

tggaactcag gcgccctgac cagcggcgtg cacaccttcc cggctgtcct acagtcctca     180

ggactctact ccctcagcag cgtggtgacc gtgccctcca gcagcttggg cacccagacc     240

tacatctgca acgtgaatca caagcccagc aacaccaagg tggacaagaa agttgagccc     300

aaatcttgtg acaaaactca cacatgccca ccgtgcccag cacctgaact cctgggggga     360

ccgtcagtct tcctcttccc cccaaaaccc aaggacaccc tcatgatctc ccggacccct     420

gaggtcacat gcgtggtggt ggacgtgagc cacgaagacc ctgaggtcaa gttcaactgg     480

tacgtggacg gcgtggaggt gcataatgcc aagacaaagc cgcgggagga gcagtacgcc     540

agcacgtacc gtgtggtcag cgtcctcacc gtcctgcacc aggactggct gaatggcaag     600

gagtacaagt gcaaggtctc caacaaagcc ctcccagccc ccatcgagaa aaccatctcc     660

aaagccaaag ggcagccccg agaaccacag gtgtacaccc tgcccccatc ccgggatgag     720

ctgaccaaga accaggtcag cctgacctgc ctggtcaaag gcttctatcc cagcgacatc 780

gccgtggagt gggagagcaa tgggcagccg gagaacaact acaagaccac gcctcccgtg 840

ctggactccg acggctcctt cttcctctac agcaagctca ccgtggacaa gagcaggtgg 900

cagcagggga acgtcttctc atgctccgtg atgcatgagg ctctgcacaa ccactacacg 960

cagaagagcc tctccctgtc tccgggtaaa tga 993